# EUROPEAN PATENT SPECIFICATION

(11) **EP 4 568 716 B1**
(45) Date of publication and mention of the grant of the patent: **05.08.2026**
(21) Application number: 23777425.2
(22) Date of filing: 01.09.2023
(51) Int. Cl.: A61L 27/50, A61L 31/02, A61L 31/18, A61L 27/06

(54) **IMPROVED RADIOPACITY IN IMPLANTABLE MEDICAL DEVICES**
VERBESSERTE STRAHLUNGSUNDURCHLÄSSIGKEIT IN IMPLANTIERBAREN MEDIZINISCHEN VORRICHTUNGEN
RADIO-OPACITÉ AMÉLIORÉE DANS DES DISPOSITIFS MÉDICAUX IMPLANTABLES

(30) Priority: 02.09.2022 US 202263374480 P
(43) Date of publication of application: 18.06.2025
(73) Proprietor: Edwards Lifesciences Corporation, Irvine, CA 92614 (US)
(72) Inventor: LAUNEY, Maximilien, Evans, Pasadena, California 91101 (US); WU, Ming, H., Tustin, California 92782 (US); CAO, Hengchu, Irvine, California 92614 (US); SCHNEIDER, Ralph, Irvine, California 92614 (US); SHARMA, Nitin, Irvine, California 92614 (US)
(74) Representative: Venner, Julia Ann
(86) International application number: PCT/US2023/031898
(87) International publication number: WO 2024/050118

(56) References cited:
- WO-A2-01/74274
- US-A1- 2008 027 532
- US-B1- 10 076 403
- CHRISTOPH BECHTOLD ET AL: "Method for Fabricating Miniaturized NiTi Self-Expandable Thin Film Devices with Increased Radiopacity", SHAPE MEMORY AND SUPERELASTICITY, vol. 2, no. 4, 21 November 2016 (2016-11-21), pages 391 - 398, XP055573835, ISSN: 2199-384X, DOI: 10.1007/s40830-016-0086-8

## Description

### CROSS-REFERENCE TO RELATED APPLICATION(S)

This application claims the benefit of U.S. Provisional Application No. 63/374,480, filed September 2, 2022, and entitled "IMPROVED RADIOPACITY IN IMPLANTABLE MEDICAL DEVICES."

### BACKGROUND

This application relates to radiopaque implantable medical devices, and, more particularly, to using physical vapor deposition to form radiopaque implantable medical devices.

Fluoroscopy and other X-ray-based visualization techniques are used to determine the anatomical location and orientation of implantable devices within a patient's body. X-ray based visualization techniques aid in medical procedures, including, for example, implantation of stents, shunts, and replacement heart valves. In many instances, the super elastic materials necessary for implantable devices have inherently low radiopacity (low X-ray absorption). Traditionally, radiopaque markers are fixed to the devices during implantation to visualize the devices' positions. However, the markers are small and provide only the position of the marker rather than the entire body of the device to which the markers are attached. It is desirable to visualize the entirety of a medical device to ensure proper placement and orientation within a patient.

US 10 076 403 B1 discloses a shunt for regulating blood pressure between a patient's left atrium and right atrium. The shunt comprises a body comprising struts and openings between the struts. In a preferred embodiment, the device comprises an anchor configured to be implanted in the interatrial septum and a conduit affixed to the anchor. The anchor comprises a self-expanding material, such as a shape memory alloy, and circumferential struts. The anchor may comprise a biocompatible metal framework or laser-cut solid metallic tube made from a list including nitinol and titanium alloy. The anchor includes elongated portions and eyelets that extend into the right atrium when the shunt is employed. In accordance with one aspect of the invention, alternating eyelets include radiopaque markers, for example made of platinum, iridium, gold, tantalum, or any other similar suitable material, which enhance visualization of the shunt under fluoroscopy.

### SUMMARY

The scope of protection is defined by the claims.

Also disclosed herein, but not part of the claimed invention, is a method of manufacturing an implantable medical device with radiopaque properties, the method including converting an ingot of a metal alloy with radiopaque and super elastic properties into a rod. The method also includes drilling the rod to create a hollow tube. The method also includes drawing the tube. The method also includes manufacturing an implantable medical device form from the tube. The method also includes finishing the implantable medical device form to create an implantable medical device.

In one aspect, the presently claimed invention relates to an implantable medical device with radiopaque properties according to claim 13, wherein the implantable device is a shunt, wherein the shunt comprises a body comprising struts and openings between the struts. The implantable medical device is manufactured using a physical vapor deposition process. The implantable medical device is made from a nickel-titanium-platinum (NiTiPt) alloy.

### BRIEF DESCRIPTION OF THE DRAWINGS

### PVD DEVICES AND PROCESSES

FIG. 1 is a schematic of a physical vapor deposition (PVD) device.
FIG. 2 is a schematic of an alternate PVD device.
FIG. 3A is a schematic of a vacuum chamber during an arc-evaporation PVD process.
FIG. 3B is a schematic of a vacuum chamber during an ion beam evaporation PVD process.
FIG. 4 is a schematic of a vacuum chamber during a sputtering PVD process.

### IMPLANTABLE MEDICAL DEVICE 60

FIG. 5 is a schematic cross section of a first example of an implantable medical device.
FIG. 6 is a flow chart showing a method for manufacturing the first example of the implantable medical device.
FIG. 7 is a graph showing predicted radiopacity improvement in the first example of the implantable medical device.

### IMPLANTABLE MEDICAL DEVICE 90

FIG. 8 is a schematic cross section of a second example of an implantable medical device.
FIG. 9 is a flow chart showing a method for creating the second example of the implantable medical device.
FIG. 10 is a graph showing radiopacity improvement in the second example of the implantable medical device.

### IMPLANTABLE MEDICAL DEVICE 120

FIG. 11 is a schematic cross section of a third example of an implantable medical device.
FIG. 12A is a flow chart showing a method of using PVD to create the third example of the implantable medical device.
FIG. 12B is a flow chart showing a method of using wrought methods to create the third example of the implantable medical device.
FIG. 13 is a fluoroscopic image comparing radiopacity of different alloy compositions .
FIG. 14 is a graph showing radiopacity improvement in the third example of the implantable medical device.

### NiTiPt ALLOYS: ALLOY 200A, ALLOY 200B, AND ALLOY 200C

FIG. 15 is a fluoroscopic image showing radiopacity improvement in three NiTiPt alloys.
FIG. 16 is a scatterplot showing radiopacity improvement in the three NiTiPt alloys over binary NiTi.
FIG. 17 is a scatterplot showing ingot As temperatures for the three NiTiPt alloys using different cooling rates.
FIG. 18 is a scatterplot showing ingot As temperatures for the three NiTiPt alloys after heat treatment and aging at different temperatures.
FIG. 19 is a graph showing wire tensile curves for the three NiTiPt alloys between 0 GPa and 1.6 GPa of stress applied.

### IMPLANTABLE MEDICAL DEVICE 260

FIG. 20A is a schematic cross section of a fourth example of an implantable medical device.
FIG. 20B is a fluoroscopic image of three samples of wire on a model torso.
FIG. 20C is a fluoroscopic image of three samples of wire and two sample implantable medical devices on a model torso.
FIG. 20D is a fluoroscopic image of three samples of wire and two sample implantable medical devices in a model torso.

### HEART H AND VASCULARTURE V

FIG. 21 is a schematic diagram of a heart and vasculature.
FIG. 22 is a cross-sectional view of the heart.

### SHUNT DEVICE 300

FIG. 23A is a perspective view of a shunt device.
FIG. 23B is a side view of the shunt device.
FIG. 23C is a bottom view of the shunt device.
FIG. 24 is a perspective view of the shunt device in a collapsed configuration.
FIG. 25 is a side view of the shunt device with a sensor and anchored to a tissue wall.

### CARDIOVASCULAR IMPLANT DEVICE 400

FIG. 26A is a perspective view of a first example of a cardiovascular implant device.
FIG. 26B is a sectional view of a heart illustrating an example positioning at a non-valve site of the first example of the cardiovascular implant device.

### CARDIOVASCULAR IMPLANT DEVICE 500

FIG. 27A is a perspective view of a second example of a cardiovascular implant device.
FIG. 27B is a sectional view of a heart illustrating an example positioning at a valve site of the second example of the cardiovascular implant device.

### CARDIOVASCULAR IMPLANT DEVICE 600

FIG. 28 is a perspective view of a third example of a cardiovascular implant device.
FIG. 29 is a sectional view of a heart illustrating an example positioning at a valve site of the third example of the cardiovascular implant device.
FIG. 30 is a sectional view of a heart illustrating an example positioning at a non-valve site of the third example of the cardiovascular implant.

### CARDIOVASCULAR IMPLANT DEVICE 700

FIG. 31 is a sectional view of a heart illustrating an example positioning of a fourth example of a cardiovascular implant device.

### CARDIOVASCULAR IMPLANT DEVICE 800

FIG. 32 is a sectional view of a heart illustrating an example positioning of a fifth example of a cardiovascular implant device.

### DETAILED DESCRIPTION

In general, this disclosure relates to improving radiopacity of implantable medical devices by manufacturing the implantable medical device out of materials with high radiopacity and biocompatible super elastic and/or shape-memory materials. Implantable medical devices manufactured out of both materials show improved radiopacity while maintaining super elasticity and biocompatibility. The implantable medical devices of the disclosure can be created using three approaches (not part of the claimed invention): applying a coating or a layer of a radiopaque material to an existing implantable medical device, manufacturing an implantable device out of a multi-component alloy system (for example, ternary and quaternary alloys) with both super elastic and radiopaque properties, and a composite materials approach. Applying a coating or a layer of a radiopaque material to an implantable medical device can include using physical vapor deposition (PVD) to form the coating or the layer on implantable medical devices. The composite materials approach includes manufacturing an implantable medical device out of a composite material with a radiopaque core inside a super elastic sheath.

The implantable medical devices of the presently claimed invention are created by manufacturing an implantable device out of a multi-component, ternary alloy system with both super elastic and radiopaque properties. Manufacturing an implantable medical device out of a ternary alloy includes creating an alloy with high radiopacity and super elastic properties that is then processed using PVD to create an implantable medical device.

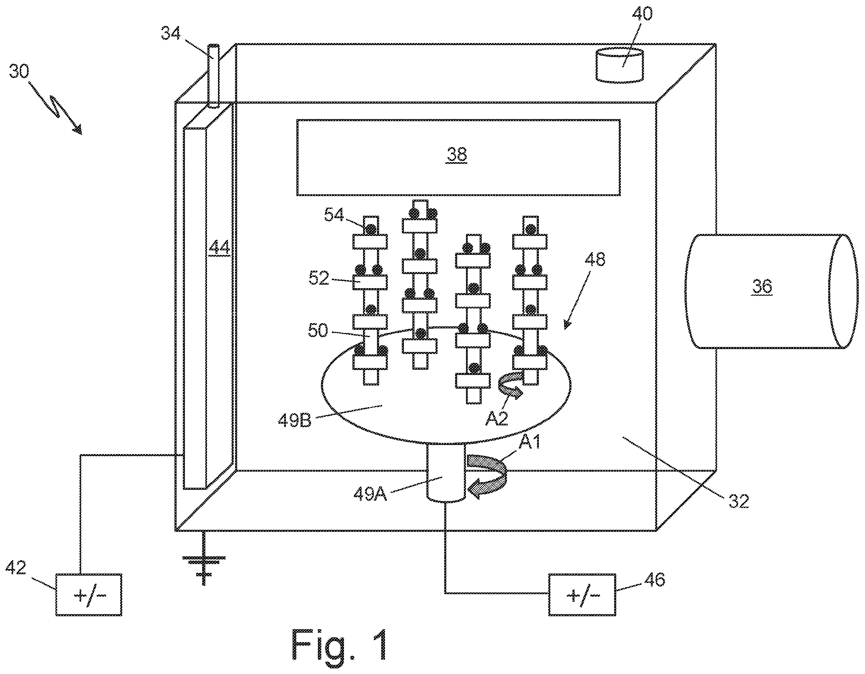

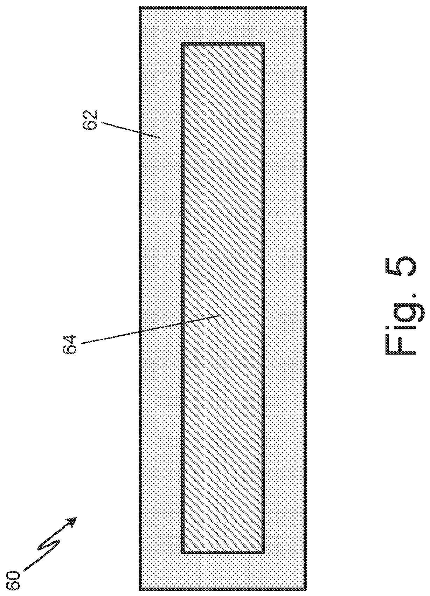

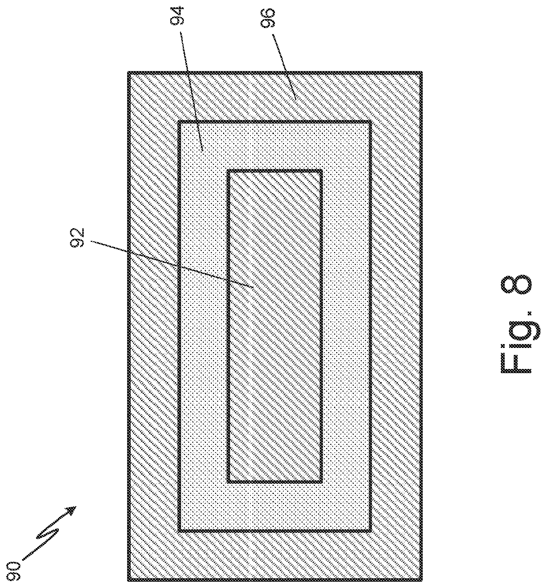

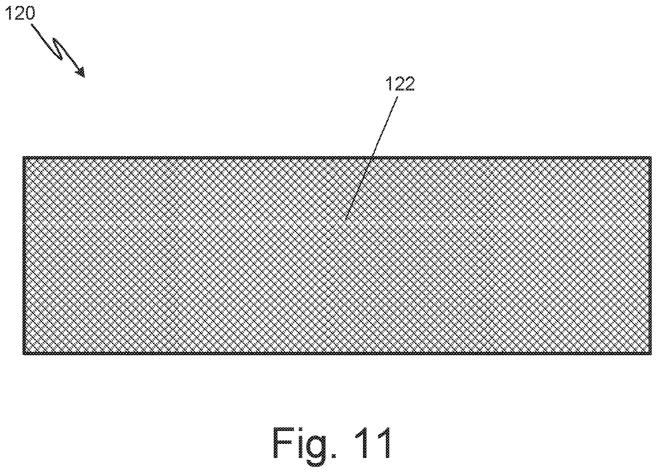

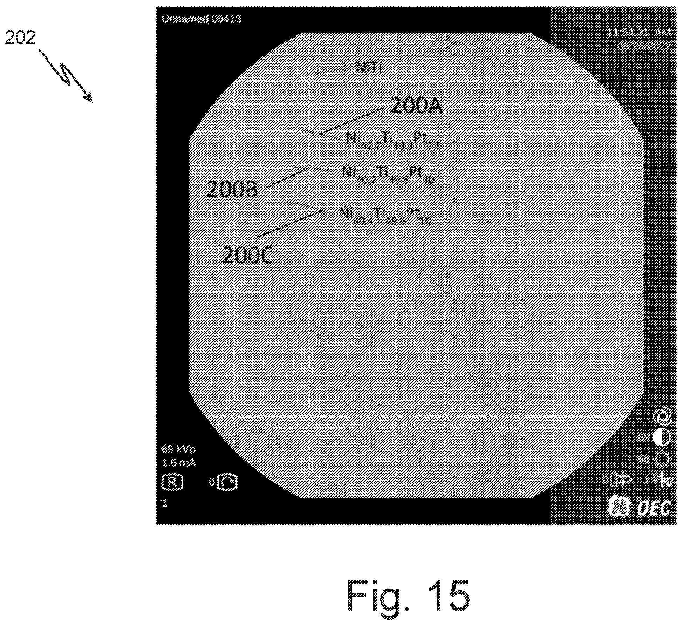

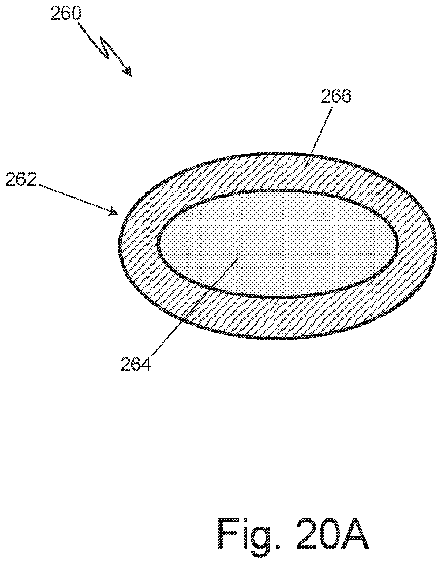

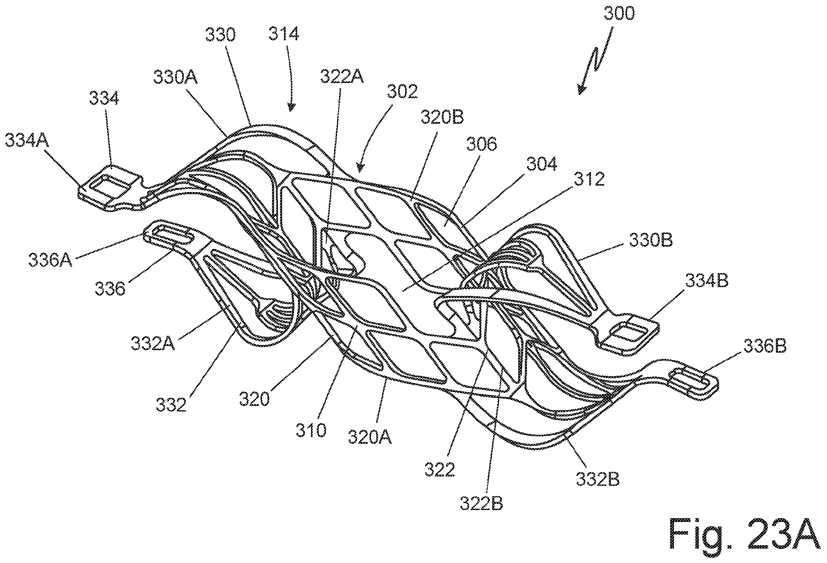

This disclosure will begin by describing PVD devices and methods, as described in relation to FIGS. 1-4. The PVD methods described include arc-evaporation PVD, ion-beam evaporation PVD, magnetron sputtering PVD, and ion-beam sputtering PVD. An implantable medical device having a radiopaque coating and a method of applying a radiopaque coating to an implantable medical device using a PVD process is described with respect to FIGS. 5-7 ( not part of the claimed invention). An implantable medical device having a radiopaque layer and a method of applying a radiopaque layer and a super elastic layer to an implantable medical device using a PVD process is described with respect to FIGS. 8-10 (not part of the claimed invention). An implantable medical device made from a ternary or quaternary alloy and a method of manufacturing an implantable medical device from a ternary or quaternary alloy is described with respect to FIGS. 11-14. Example NiTiPt alloys that can be used in implantable medical devices are described in relation to FIGS. 15-19. An implantable medical device made from a composite material is described with respect to FIG. 20A-20D (not part of the claimed invention). Next, the disclosure will describe implantable medical devices made from the super elastic materials with high radiopacity, as described in relation to FIGS. 23A-32. Implantable medical devices include shunts ( part of the claimed invention) as well as stents ( not part of the claimed invention) and docking stations (not part of the claimed invention).

### PVD DEVICES AND PROCESSES

FIG. 1 is a schematic of physical vapor deposition (PVD) device 30. PVD device 30 includes vacuum chamber 32 with inlet 34, outlet 36, heater 38, and vacuum sensor 40. PVD device 30 also includes power supply system 42, target 44, bias power supply system 46, and rotating carousel 48 with standoff 49A, platform 49B, vertical supports 50 and shelves 52. FIG. 1 also shows substrate forms 54, arrow A1, and arrow A2.

PVD device 30 has vacuum chamber 32 that is grounded. Inlet 34 connects to a top of vacuum chamber 32. Inlet 34 connects to a gas source (not shown in FIG. 1). Outlet 36 connects to a first side of vacuum chamber 32. Outlet 36 connects to a vacuum (not shown in FIG. 1). Heater 38 is inside vacuum chamber 32 between inlet 34 and outlet 36. Vacuum chamber 32 also connects to vacuum sensor 40, which is at a top of vacuum chamber 32 in FIG. 1. However, vacuum sensor 40 could be located at other positions in vacuum chamber 32. Power supply system 42 enters at a second side of vacuum chamber 32 across from outlet 36. Target 44 is mounted on the second side of vacuum chamber 32 and is connected to power supply system 42. Bias power supply system 46 is near a bottom of vacuum chamber 32 and connects to rotating carousel 48. Rotating carousel 48 is in a center of vacuum chamber 32 and includes standoff 49A and platform 49B. Multiple vertical supports 50 are positioned on the platform 49B of rotating carousel 48 and each vertical support 50 has multiple shelves 52. Shelves 52 each support substrate forms 54. Substrate forms 54 can be implantable medical devices or forms that can be used to manufacture implantable medical devices. Rotating carousel 48 and vertical supports 50 are configured to rotate. In FIG. 1, arrow A1 indicates that carousel 48 rotates clockwise and arrow A2 indicates that vertical supports 50 rotate counterclockwise. Rotating carousel 48, vertical supports 50, shelves 52, and substrate forms 54 are located between target 44 and outlet 36.

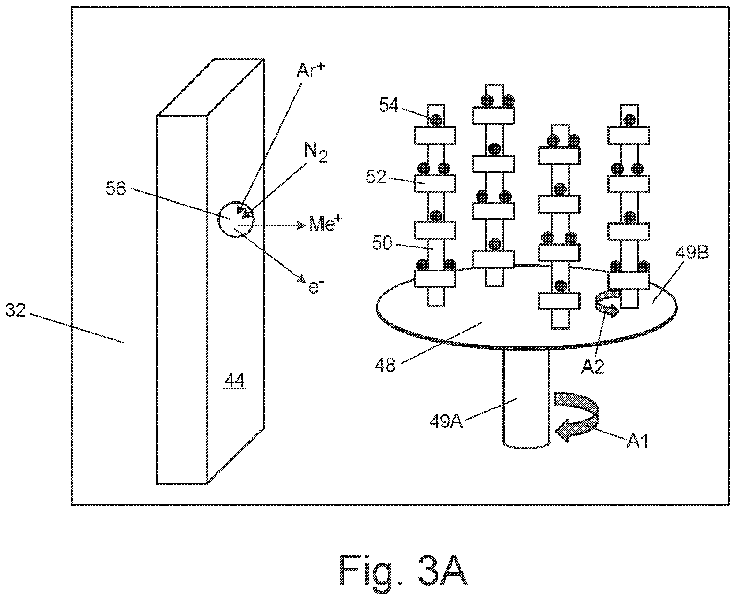

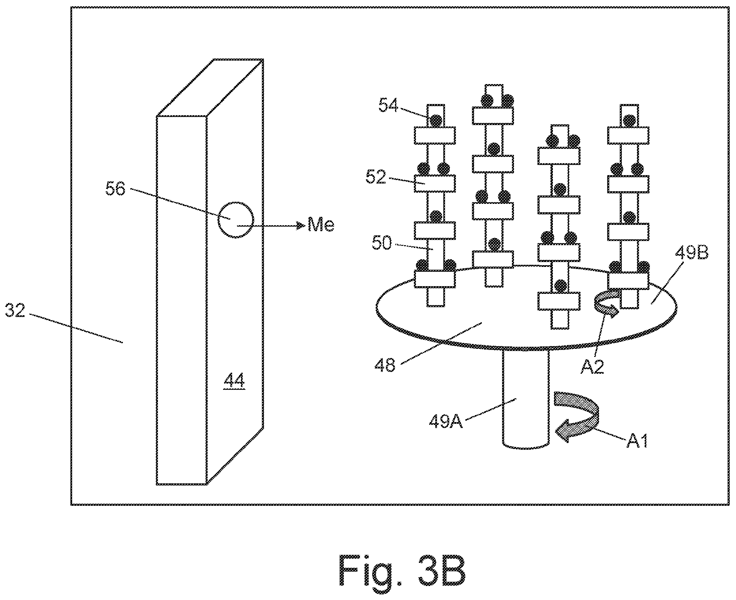

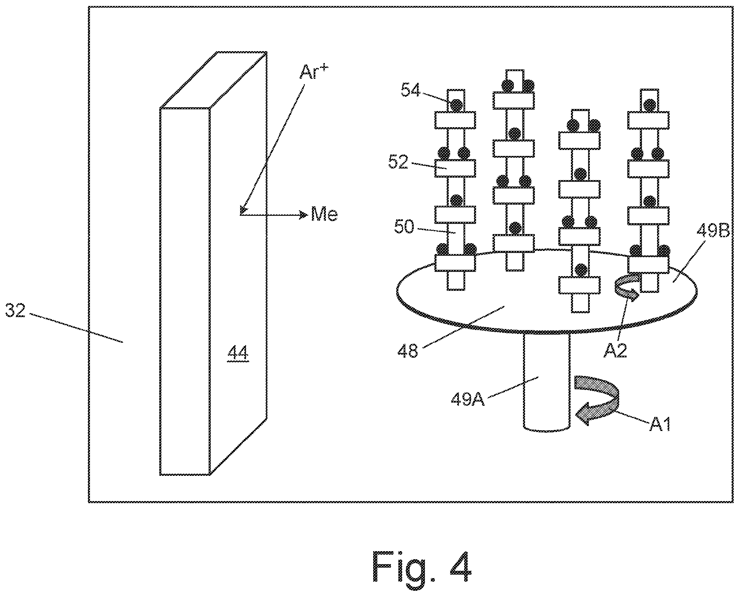

PVD device 30 can be used for PVD processes, including, for example, evaporation processes and sputtering processes. Evaporation processes can include arc-evaporation and ion-beam evaporation. Evaporation processes vaporize a target material (for example target 44) to coat a substrate (for example substrate forms 54). Sputtering processes can include magnetron sputtering and ion-beam sputtering. Sputtering processes collide energetic ions against a target material (for example target 44) to eject target material to coat a substrate (for example substrate forms 54). Evaporation processes, including arc-evaporation PVD and ion-beam evaporation PVD, will be discussed in detail with respect to FIG. 3A and 3B. Sputtering processes, including magnetron sputtering PVD and ion-beam sputtering, will be discussed in detail with respect to FIG. 4.

Generally, PVD processes take place under vacuum, which is created in vacuum chamber 32. Outlet 36 is connected to a vacuum to remove air, carrier gas, excess process gas, spent process gas, and excess material created during PVD processes from vacuum chamber 32. Heater 38 warms vacuum chamber 32 to temperatures necessary for PVD processes. Vacuum sensor 40 monitors pressure within vacuum chamber 32. Once vacuum chamber 32 is under vacuum and heated, inlet 34 can be used to introduce process gasses (for example energetic ions) and carrier gasses (for example dinitrogen) to vacuum chamber 32. Power supply system 42 is used to charge target 44 appropriately for the PVD process being used. Process gases then interact with charged target 44 to eject metal ions. Bias power supply system 46 creates the proper charge on rotating carousel 48, vertical supports 50, shelves 52, and substrate forms 54 to attract the ejected metal ions. A thin layer of metal from the ejected metal ions collects on each of substrate forms 54. PVD device 30 can coat very small implantable medical devices, including those with lengths of less than five inches . PVD device 30 can be used to coat substrate 54 with coatings of material, for example, between 7 micrometers and 25 micrometers thick. PVD device 30 can also be used to form an implantable medical device (either a complete device or a component of an implantable medical device assembly) out of a radiopaque alloy that is between, for example, 50 micrometers and 250 micrometers thick. More specifically, PVD device 30 can be used to form an implantable medical device (either a complete device or a component of an implantable medical device assembly) out of a radiopaque alloy that is between, for example, 100 micrometers and 150 micrometers thick.

PVD device 30 can be used to apply a coating or a layer of radiopaque material on an implantable medical device or to create an implantable medical device out of an alloy having radiopaque and super elastic properties. Radiopaque materials tend to be expensive materials. Using PVD device 30 to create a thin layer of metal on substrate forms 54 is an economical way to create a medical device from or coat a medical device with an expensive substance, for example platinum. Target 44 requires a relatively small amount of material to create a layer between 7 micrometers and 15 micrometers thick on an implantable medical device. PVD processes undergone in PVD device 30 result in less weight than traditional manufacturing methods, increasing economic efficiency. Rotating carousel 48 increases uniformity in coating substrate forms 54.

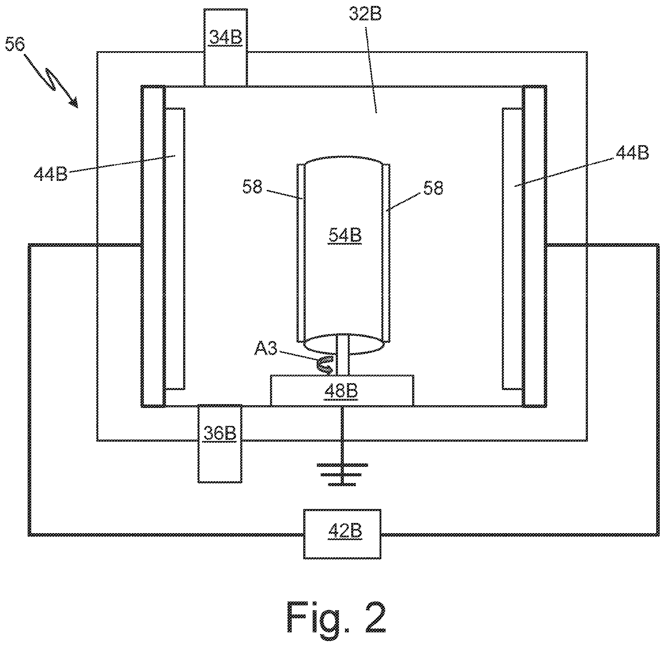

FIG. 2 is a schematic of alternate PVD device 56. PVD device 56 includes vacuum chamber 32B with inlet 34B and outlet 36B, power supply system 42B, targets 44B, and rotating carousel 48B. FIG. 2 also shows substrate form 54B, deposited material 58, and arrow A3. PVD device 56 has a similar structure as PVD device 30 shown in FIG. 1. Components of PVD device 56 that are similar to components of PVD device 30 have been labeled with similar reference numbers having a "B" added to the number. For example, vacuum chamber 32B in FIG. 2 is similar to vacuum chamber 32 in FIG. 1.

PVD device 56 has vacuum chamber 32B with inlet 34B across from outlet 36B. Inlet 34B is in a top of vacuum chamber 32B, and outlet 36B is in a bottom of vacuum chamber 32B. Inlet 34B is connected to a tank holding process gas and/or carrier gas (not shown in FIG. 2). Outlet 36B is connected to a vacuum (not shown in FIG. 2). Power supply system 42B includes plates within vacuum chamber 32B. The plates of power supply system 42B are on opposing sides of vacuum chamber 32B. Targets 44B are adjacent to the plates of power supply system 42B. Near a middle of vacuum chamber 32B is rotating carousel 48B, which is grounded. Rotating carousel 48B holds substrate form 54B. Substrate form 54B can be an implantable medical device or a form that can be used to manufacture implantable medical devices. Deposited material 58 is shown on opposing sides of substrate form 54B. However, as PVD processing continues, deposited material can cover substrate form 54B. Covering substrate form 54B is aided by rotating carousel 48B. Arrow A3 indicates that rotating carousel 48B rotates counterclockwise. Rotating carousel 48B can alternatively rotate clockwise.

PVD device 56 can be used for PVD processes, including, for example, arc-evaporation, ion-beam evaporation, magnetron sputtering, and ion-beam sputtering. Evaporation PVD vaporizes a target material (for example target 44B) to coat a substrate (for example substrate form 54B). Sputtering PVD collides energetic ions against a target material (for example target 44B) to eject the target material to coat a substrate (for example substrate forms 54B). Evaporation PVD and sputtering PVD will be discussed in detail with respect to FIG. 3 and FIG. 4, respectively.

PVD device 56 operates similarly to PVD device 30 with respect to vacuum chamber 32B, inlet 34B, outlet 36B, and power supply system 42B. However, PVD device 56 has targets 44B in multiple locations within vacuum chamber 32B, rotating carousel 48B holds one substrate form 54B. Substrate form 54B can be, for example, a binary nickel-titanium alloy (nitinol) tube or medical device. Substrate form 54B includes deposited layer 58. Targets 44B are shown on opposite sides of vacuum chamber 32B, however targets 44B can be located in multiple locations of vacuum chamber 32B depending on how power supply system 42B is connected to vacuum chamber 32B. PVD device 56 also includes a different configuration for rotating carousel 48B compared to rotating carousel 48 shown in FIG. 1. Rotating carousel 48B holds a single substrate form (substrate form 54B in FIG. 2). Multiple rotating carousels 48B each holding a substrate forms 54B can be placed into reaction chamber 32B. Reaction chamber 56 includes deposited material 58 on opposing sides of substrate form 54B. Material from targets 44B is evaporated and deposited onto substrate 54B. As such, targets 44B and substrate 54B are the same material. Deposited material 58 and targets 44B can be made from metals and metal alloys. PVD device 56 can be used for multiple types of PVD processes including arc-evaporation PVD (as will be discussed in relation to FIG. 3) and magnetron sputtering PVD (as will be discussed in relation to FIG. 4). PVD device 56 can coat very small implantable medical devices, including those with lengths of less than 5 inches. PVD device 30 can be used to coat substrate 54 with coatings of material, for example, between 7 micrometers and 25 micrometers thick. PVD device 30 can also be used to form an implantable medical device (either a complete device or a component of an implantable medical device assembly) out of a radiopaque alloy that is between, for example, 50 micrometers and 250 micrometers thick. More specifically, PVD device 30 can be used to form an implantable medical device (either a complete device or a component of an implantable medical device assembly) out of a radiopaque alloy that is between, for example, 100 micrometers and 150 micrometers thick. PVD device 56 has similar benefits as PVD device 30. PVD device 56 can be used to form a coating or a layer of radiopaque material on an implantable medical device to create an implantable medical device out of a radiopaque material. Radiopaque materials tend to be expensive materials. PVD device 56 can be an economical tool to coat small implantable medical devices with thin layers of radiopaque materials (typically coatings are between 7 micrometers and 15 micrometers). This economic benefit is especially true when using high-value radiopaque materials like platinum and gold. PVD device 56 requires very little material to create targets 44B. Rotating carousel 48B increases uniformity in coating substrate form 54B. Having targets 44B in multiple locations within vacuum chamber 32B increases the speed of a PVD process and regularity of products from the process.

FIG. 3A is a schematic of vacuum chamber 32 during an arc-evaporation PVD process. FIG. 3A shows vacuum chamber 32 with target 44, and rotating carousel 48. Rotating carousel 48 includes standoff 49A and platform 49B. Vertical supports 50, shelves 52, and substrate forms 54 are positioned on rotating carousel 48. FIG. 3A also includes cathode spot 56, argon cations Ar⁺, dinitrogen N₂, metal cations Me⁺, and electrons e⁻. Arc-evaporation processes like those illustrated in FIG. 3A can be undergone in any suitable PVD device, for example PVD device 30 discussed in relation to FIG. 1 or PVD device 56 discussed in relation to FIG. 2.

Vacuum chamber 32 is the same in FIG. 3A as in FIG. 1 with respect to rotating carousel 48 with standoff 49A and platform 49B, vertical supports 50, shelves 52, and substrate forms 54. However, target 44 includes cathode spot 56, which is a point of molten metal on target 44. Argon cations Ar⁺ and dinitrogen N₂ have arrows representing interactions with cathode spot. Metal cations Me⁺ and electrons e⁻ are being released from cathode spot.

Arc-evaporation PVD is one way to vaporize target 44. In arc-evaporation PVD, as shown in FIG. 3A, target 44 is a cathode. Arc-evaporation PVD strikes target 44 with a high current, low voltage arc at a surface target 44. Power supply system 42 (shown in FIG. 1) can be used to create the arc that strikes target 44. Cathode spot 56 forms where the arc strikes target 44. Cathode spot 56 is molten metal and can reach temperatures around 15,000°C. High temperatures at cathode spot 56 result in a high velocity jet of vaporized material from target 44. The jet can reach speeds of 10 kilometers per second. Introduction of a reactive gas, like dinitrogen N₂ or argon cations Ar⁺, occurs through inlet 34 to reaction chamber 32. Introduced reactive gasses cause dissociation, ionization, and excitation in target 44, resulting in free metal cations Me⁺ and electrons e⁻ within vacuum chamber 32. Metal cations can have charges of +1, +2, +3, or others depending on the metal used to make target 44. Metal cations Me⁺ and electrons e⁻ deposit onto substrate forms 54 creating a thin layer of material with the chemical makeup of target 44. A magnetic field around target 44 moves cathode spot 56 to evenly vaporize target 44. For arc-evaporation PVD, target44 must be conductive. Arc-evaporation creates a hard, durable coating with a relatively short cycle time.

FIG. 3B is a schematic of vacuum chamber 32 during an ion-beam evaporation PVD process. FIG. 3B shows vacuum chamber 32 with target 44, and rotating carousel 48. Rotating carousel 48 includes standoff 49A and platform 49B. Vertical supports 50, shelves 52, and substrate forms 54 are positioned on rotating carousel 48. FIG. 3B also includes molten spot 56 and metal atoms Me. Ion-beam evaporation processes like those illustrated in FIG. 3B can be undergone in any suitable PVD device, for example PVD device 30 discussed in relation to FIG. 1 or PVD device 56 discussed in relation to FIG. 2.

Vacuum chamber 32 shown in FIG. 3B is the same as in FIG. 1 with respect to rotating carousel 48 with standoff 49A and platform 49B, vertical supports 50, shelves 52, and substrate forms 54. However, target 44 includes molten spot 56, which is a point of molten metal on target 44. Metal atom Me is being evaporated from molten spot 56.

Ion beam evaporation PVD is another way to vaporize target 44. In ion beam evaporation, a beam of ions is used to evaporate target 44. The ion beam can be aimed across the surface of target 44 to evenly vaporize target 44. Vaporized metal Me are metal atoms that are sufficiently energized to become gaseous and migrate toward substrate forms 54. Ion beam evaporation utilizes an ion beam which can be adjustable to provide proper heat to vaporize target 44. Using ion beam evaporation also allows for non-conducting materials to be target 44.

FIG. 4 is a schematic of vacuum chamber 32 during a sputtering PVD process. FIG. 4 shows vacuum chamber 32 with target 44, and rotating carousel 48. Rotating carousel 48 includes standoff 49A and platform 49B. Vertical supports 50, shelves 52, and substrate forms 54 are positioned on rotating carousel 48. FIG. 4 also includes argon cations Ar⁺ and metal atoms Me. Sputtering processes like those illustrated in FIG. 4 can be undergone in any suitable PVD device, for example PVD device 30 discussed in relation to FIG. 1 or PVD device 56 discussed in relation to FIG. 2.

Vacuum chamber 32 is the same in FIG. 4 as in FIG. 1 with respect to rotating carousel 48, vertical supports 50, shelves 52, and substrate forms 54. However, vacuum chamber 32 in FIG. 4 includes argon cations Ar⁺ which bombard a surface of target 44 to eject metal atoms Me .

Magnetron sputtering PVD is one sputtering method. In magnetron sputtering PVD, target 44 is bombarded with ions from a plasma, atoms of target 44 are ejected, and then atoms are deposited on substrate 54 to create a layer of the target 44 material on substrate 54. Specifically, a power supply system (like power supply system 42 shown in FIG. 1) applies a negative voltage to target 44. The negative voltage can be around -300V or more. Vacuum chamber 32 is then charged with a non-reactive gas, which is argon in this example. Argon moving near negatively charged target 44 will be stripped of electrons to form argon cations Ar⁺ in plasma form. The negatively charged target 44 attracts argon cations Ar⁺ from the plasma. Argon cations Ar⁺ bombard the surface of target 44 and eject metal atoms Me from the surface of target 44. Metal atoms Me deposit onto substrate forms 54 in vacuum chamber 32 creating a thin coating. The presence of plasma can clean substrate 54 and increase adhesion of material from target 44.

Ion-beam sputtering PVD is another sputtering method. Ion-beam sputtering is similar to magnetron sputtering. In ion-beam sputtering PVD, target 44 is bombarded with ions from an ion source, atoms of target 44 are ejected, and then atoms are deposited on substrate 54 to create a layer of the target 44 material on substrate 44. Ions, for example argon cations Ar⁺, are created by an ion source, collimated, and aimed at target 44 using, for example, an ion gun. The ions produced by an ion source like an ion gun can be directed, have energies ranging from a few eV to 1,000 eV, and have multiple configurations. In ion-beam sputtering, the argon cations Ar⁺ (ions) are directed toward a surface of target 44. Argon cations Ar⁺ bombard the surface of target 44 and eject metal atoms Me from the surface of target 44. Metal atoms Me deposit onto substrate forms 54 in vacuum chamber 32 creating a thin coating. Ion-beam sputtering does not utilize a plasma, which allows for depositing sensitive substrates and lowers gas deposition on the substrate 54, leading to a denser layer of target 44 material deposition. Further, no energetic bias is needed in ion-beam sputtering, so conducting and non-conducting targets 44 can be used.

Arc-evaporation PVD discussed with respect to FIG. 3A, ion beam evaporation PVD discussed with respect to FIG. 3B, magnetron sputtering PVD discussed with respect to FIG. 4, and ion beam sputtering PVD, discussed with respect to FIG. 4 are PVD processes that can be used to create different radiopaque coatings or layers on implantable medical devices or can be used to create radiopaque implantable medical devices. Radiopaque coatings, radiopaque layers, and radiopaque implantable medical devices that can be created with PVD processes will be discussed with respect to FIGS. 5-14. Alloys of NiTiPt that can be deposited using PVD processes will be discussed in relation to FIGS. 15-19. Composite implantable medical devices having radiopacity are discussed in relation to FIGS. 20A-20D. Examples of implantable medical devices that PVD process and coatings can be used with will be discussed with respect to FIGS. 23A-32.

### IMPLANTABLE MEDICAL DEVICE 60 (not part of the claimed invention)

FIG. 5 is a schematic cross-section of implantable medical device 60. FIG. 5 shows implantable medical device 60 including substrate 62 and coating 64.

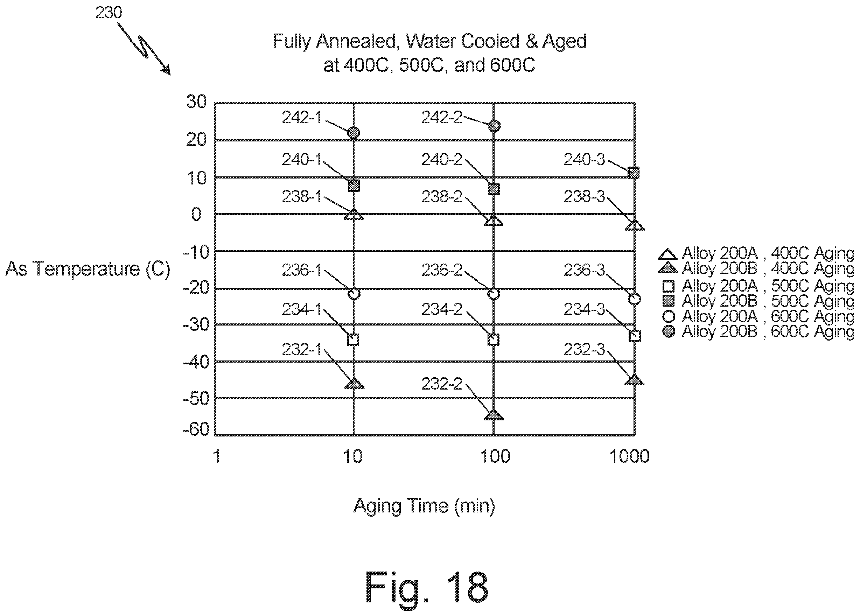

Implantable medical device 60 as shown in FIG. 5 is a schematic representation of a cross-section of an implantable medical device having substrate 62 and coating 64 on substrate 62. Implantable medical device 60 can be any implantable medical device having any shape and size. Further, the cross-section shown in FIG. 5 can be a representation of a section or portion of implantable medical device 60. In the following discussion, implantable medical device 60 will be used as an example of a device that can be made from substrate 62 and coating 64. However, implantable medical device 60 can be any suitable medical device, including any of implantable medical devices 300, 400, 500, 600, 700, and 800 shown in FIGS. 18A-27.

Substrate 62 can be made out of a super elastic material. The super elastic material can be a nickel-titanium alloy (nitinol) including a binary nickel-titanium alloy or any other super elastic or shape-memory alloy. Coating 64 is made out of a radiopaque material. The radiopaque material can be made of a biocompatible material that absorbs X-rays and/or appears clearly when using X-ray techniques such as fluoroscopy. The radiopaque material can be chosen from row six transition metals that are not radioactive, poisonous, or have melting points below body temperature. This includes palladium, platinum, gold, iridium, hafnium, tungsten, tantalum, rhenium, zirconium, and combinations thereof. In one example, implantable medical device 60 has substrate 62 made of a nickel-titanium alloy and coating 64 made out of platinum. In a second example, implantable medical device 60 has substrate 62 made of a nickel-titanium alloy and coating 64 made out of a nickel, titanium and platinum alloy. Coating 64 can also be made of any radiopaque alloy discussed in relation to implantable medical device 120 discussed in relation to FIGS. 11-14 below.

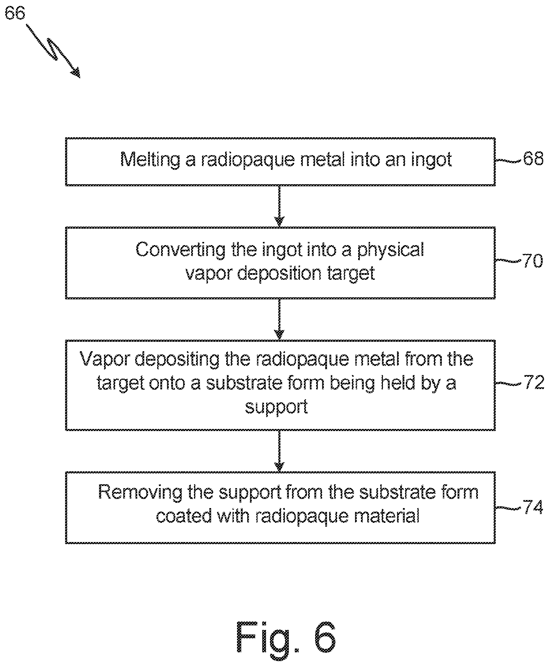

Implantable medical device 60 demonstrates both super elastic qualities and has high radiopacity. Super elasticity allows implantable medical device 60 to fit into a catheter for implantation and return to the proper shape once deployed from the catheter. The radiopaque material of coating 64 aids in placement and locating implantable medical device 60 within a patient because of its high visibility under X-ray. Further, coating 64 covers all portions of substrate 62, which improves radiopacity across the entirety of implantable medical device 60. The entire body of implantable medical device 60 will be visible under X-ray due to coating 64. Coating 64 can be added to implantable medical device 60 using PVD processes, as discussed above with respect to FIGS. 1-4. This reduces the cost of making implantable medical device 60, as will be described in more detail in relation to FIG. 6.

FIG. 6 is a flow chart showing method 66 for manufacturing implantable medical device 60. Method 66 includes step 68, step 70, step 72, and step 74.

Method 66 can be used to make implantable medical device 60. In the following discussion, implantable medical device 60 will be used as an example of a device that can be made from method 66. However, method 66 can be used to manufacture any suitable medical device, including implantable medical devices 300, 400, 500, 600, 700, and 800 shown in FIGS. 23A-32. Any suitable PVD device can be used to carry out method 66, including PVD devices 30 and 56 shown in FIGS. 1-2, respectively. Method 66 can also utilize any suitable PVD process, including arc-evaporation PVD (discussed with respect to FIG. 3), magnetron sputtering PVD (discussed with respect to FIG. 4), and ion beam PVD.

Step 68 includes melting a radiopaque material into an ingot. Step 68 can utilize any appropriate radiopaque materials for this process, including radiopaque metals such as palladium, platinum, gold, iridium, hafnium, tungsten, tantalum, rhenium, zirconium, and combinations thereof.

Step 70 includes converting the ingot into a physical vapor deposition target. One way to convert the ingot into a target is to shave a thin sheet from the ingot. Then, the sheet can be rolled into a cylinder to use as a target for PVD processes.

Step 72 includes vapor depositing the radiopaque material from the target onto a substrate form (made of substrate 62) being held by a support. Vapor depositing is done by a PVD process using a PVD device, as described, for example, in relation to FIGS 1-4. The substrate form is held by a support in the PVD device. The substrate form can be a traditional implantable medical device, for example a stent, a shunt, a replacement heart valve, a docking station, and other cardiovascular devices with struts and openings (as will be discussed in relation to FIGS. 23A-32), made of substrate 62, as discussed above with respect to FIG. 5. The substrate form can be made of super elastic or shape-memory materials, for example nitinol (e.g., binary NiTi alloy). Small substrate forms , are good candidates for method 66. Method 66 can be used to coat the substrate form with a coating that is approximately 7 micrometers to 25 micrometers thick. Small amounts of radiopaque material are deposited onto the substrate form. Method 66 coats substrate forms with approximately 7-25 micrometers of radiopaque material. Coatings (for example, coating 64) of the radiopaque material between 7 micrometers and 15 micrometers thick help retain super elastic qualities of the substrate form.

Step 74 includes removing the support from the substrate form coated with the radiopaque material. The support can be removed using a variety of processes. For example, the support may be dissolved with an acid. In another example, the support may be mechanically removed. Step 74 results in implantable medical device 60 made from substrate 62 and coating 64, as described in relation to FIG. 5.

Method 66 creates implantable medical device 60 with improved radiopacity compared to an implantable medical device made only of substrate 62 (i.e., the substrate form). Coating the substrate form with a radiopaque material allows for better visualization of the entire body of implantable medical device 60 within a patient. Using a PVD process allows a thin coating of the radiopaque material to be applied across the entire device. Coating 64 created by a PVD process increases the economic viability of using high-value radiopaque materials like platinum on implantable medical device 60. Using PVD is especially economical with high-value radiopaque materials compared to coring an ingot of material and drawing the ingot to the proper dimension for an implantable medical device. PVD processes require a small target made from a small ingot to form coating 64 of radiopaque material on substrate 62. The ability to use small amounts of material reduces material loss during coating implantable medical device 60 with the radiopaque coating 64, which makes the process more economical. PVD processes like sputtering also create plasma, which cleans the substrate form to increase adhesion. PVD processes also diffuse the radiopaque material into the substrate form, creating strong diffusion bonds between substrate 62 and radiopaque coating 64. Thin layers of radiopaque material achieved by method 66 allow the substrate form to retain super elastic characteristics.

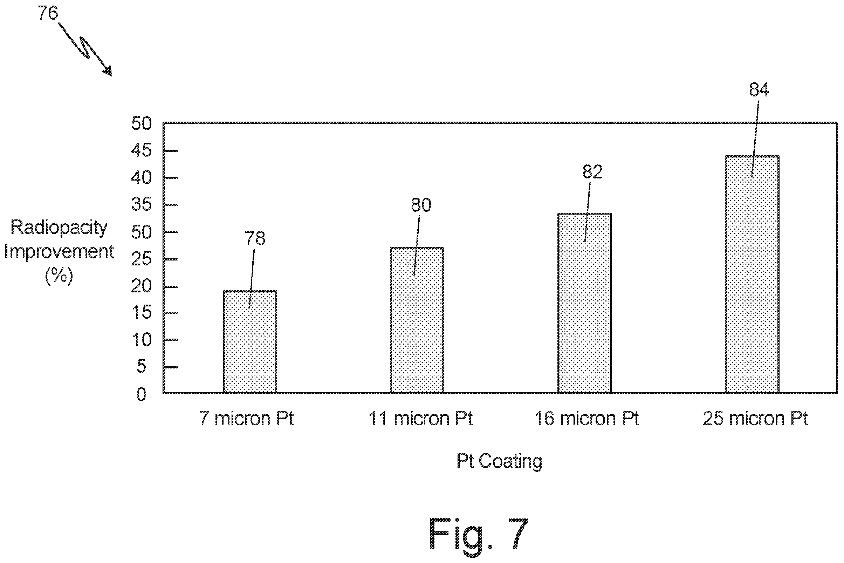

FIG. 7 is graph 76 showing predicted radiopacity improvement in implantable medical device 60 having radiopaque coating 64. Graph 76 is a bar graph with the Y-axis showing predicted radiopacity improvement in percentage compared to an implantable medical device with no radiopaque coating. The X-axis of graph 76 shows various thicknesses of platinum coatings in micrometers. Platinum coatings are one example of a radiopaque coating that can be applied to a super elastic substrate, for example nitinol (binary NiTi alloy), to form implantable medical device 60.

First bar 78 shows an approximate radiopacity improvement of 18% for a platinum coating of 7 micrometers. Second bar 80 shows an approximate improvement of 28% radiopacity improvement for a platinum coating of 11 micrometers. Third bar 82 represents an approximate radiopacity improvement of 33% for a platinum coating of 15 micrometers. Fourth bar 84 represents an approximate radiopacity improvement of 45% for a platinum coating of 25 micrometers.

Graph 76 indicates thicker coatings of platinum on a nitinol (e.g., binary NiTi alloy) device result in higher radiopacity improvement. Radiopacity improvement ranges from about 18% for a 7-micrometer layer of platinum to about 45% for a 25-micrometer coating of platinum. Increases in radiopacity means a device coated with a layer of platinum is more visible with X-ray techniques than a device without the layer of platinum. Further, as the thickness of the platinum coating increases, the radiopacity of implantable medical device 60 increases. The thickness of the platinum coating can be selected based on the level of radiopacity needed while also ensuring implantable medical device 60 retains its super elastic characteristics.

### IMPLANTABLE MEDICAL DEVICE 90 (not part of the claimed invention)

FIG. 8 is a schematic cross section of implantable medical device 90. Implantable medical device 90 includes substrate 92, radiopaque layer 94, and super elastic layer 96. Substrate 92 is a base portion of implantable medical device 90. Radiopaque layer 94 is sandwiched between substrate 92 and super elastic layer 96. Substrate 92 and super elastic layer 96 can be made from the same material, specifically nitinol (e.g., binary NiTi alloy).

Implantable medical device 90 as shown in FIG. 8 is a schematic representation of a cross-sectional of an implantable medical device having substrate 92, radiopaque layer 94, and super elastic layer 96. Implantable medical device 90 can be any implantable medical device having any shape and size. Further, the cross-section shown in FIG. 8 can be a representation of a section or portion of implantable medical device 90. In the following discussion, implantable medical device 90 will be used as an example of a device that can be made from substrate 92, radiopaque layer 94, and super elastic layer 96. However, implantable medical device 90 can be any suitable medical device, including any of implantable medical devices 300, 400, 500, 600, 700, and 800 shown in FIGS. 23A-32.

Substrate 92 can be made of a super elastic material. Substrate 92 and super elastic layer 96 can be made from nitinol (e.g., a binary NiTi alloy) or any other super elastic or shape-memory alloy. Substrate 92 and super elastic layer 96 can be made of the same material or different materials. Radiopaque layer 94 can be made of any material that absorbs X-rays and/or appears when using X-ray techniques such as fluoroscopy. Radiopaque layer 94 can be made of metals chosen from row six transition metals that are not radioactive, poisonous, or have melting points below body temperature. This includes palladium, platinum, gold, iridium, hafnium, tungsten, tantalum, rhenium, zirconium, and combinations thereof. In one example, implantable medical device 90 can have substrate 92 and super elastic layer 96 made from a nickel titanium alloy and radiopaque layer 94 made from hafnium. In a second example, radiopaque layer 94 can be made from a nickel, titanium, and platinum alloys. Radiopaque layer 94 can also be made of any radiopaque alloy discussed in relation to implantable medical device 120 discussed in relation to FIGS. 11-14 below.

Implantable medical device 90 has increased radiopacity when compared to implantable medical devices without radiopaque layer 94. Substrate 92 and super elastic layer 96 are made from biocompatible materials, for example nitinol (e.g., NiTi alloys like binary NiTi alloy). Many superelastic materials have low X-ray absorption and are hard to visualize with X-ray technology when implanting or locating an implanted medical device in a patient. Radiopaque layer 94 has a high X-ray absorption and is easier to visualize in a patient. Radiopaque layer 94 extends across the entirety of implantable medical device 90 and allows visualization of all portions of implantable medical device 90. Implantable medical device 90 has an outer coating of biocompatible material (super elastic layer 96), which increases biocompatibility of implantable medical device 90 compared to devices with exposed layers of radiopaque material 94. Super elastic layer 96 increases radial bending of implantable medical device 90, which aids in moving implantable medical device 90 into and out of catheters during placement. Implantable medical device 90 has high biocompatibility because super elastic layer 96 forms an exterior of implantable medical device 90.

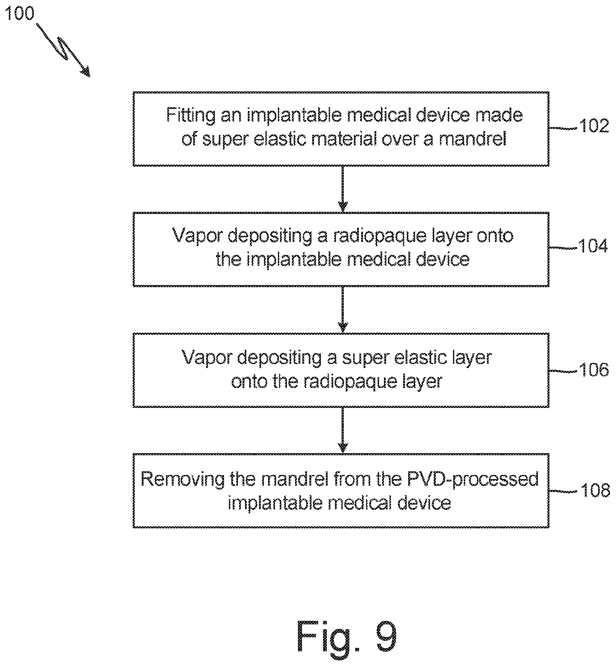

FIG. 9 is a flow chart showing method 100 for manufacturing implantable medical device 90. Method 100 includes step 102, step 104, step 106, and step 108.

Method 100 can be used to make implantable medical device 90. In the following discussion, implantable medical device 90 will be used as an example of a device that can be made from method 100. However, method 100 can be used to manufacture any suitable medical device, including any of implantable medical devices 300, 400, 500, 600, 700, and 800 shown in FIGS. 23A-32. Method 100 can also be undergone in any suitable PVD device, including PVD devices 30 and 56 shown in FIGS. 1-2, respectively. Method 100 can also utilize any suitable PVD process, including arc-evaporation PVD (discussed with respect to FIG. 3), magnetron sputtering PVD (discussed with respect to FIG. 4), and ion beam PVD. Method 100 also uses PVD targets using the same process as described in relation to steps 68 and 70 of method 66 (shown in FIG. 6). A PVD target of a radiopaque material used to form radiopaque material 94 is created following steps 68-70 as described above. Further, a PVD target of a superelastic material for use as superelastic layer 96 is created following steps 68-70 as described above.

Step 102 includes fitting an implantable medical device made of a super elastic material over a mandrel. The implantable medical device may be slightly smaller than the desired size of a finished implantable medical device to correct for thickness gained during the PVD process. The implantable medical device could be made from a nickel titanium alloy (nitinol or binary NiTi alloy) or any other biocompatible shape-memory material. The implantable medical device includes substrate 92, as described with respect to FIG. 8, and the mandrel holds the implantable medical device such that deposition of metal can occur on all surfaces of the device. The mandrel can be attached to a rotating carousel (for example rotating carousel 48 in FIG. 1 or rotating carousel 48B in FIG. 2) such that the mandrel and the implantable medical device spin and/or move about a vacuum chamber of a PVD device.

Step 104 includes vapor depositing a radiopaque layer 94 onto the implantable medical device. A PVD target of radiopaque material is made as described above in relation to steps 68-70 of method 66. In step 104, the implantable medical device is a substrate form (for example, substrate forms 54 and 54B in FIGS. 1-2). The implantable medical device can be traditional implantable medical devices, for example stents, shunts, replacement heart valves, and other cardiovascular devices with struts and openings (as will be discussed in relation to FIGS. 23A-32). The implantable medical device can be made of super elastic or shape-memory materials, for example nitinol. Small implantable medical devices are good candidates for method 100. Method 100 is more particularly useful for coating implantable medical devices with coatings between 7 micrometers and 25 micrometers thick. Small amounts of radiopaque material are deposited onto the substrate form. Method 100 coats substrate forms with approximately 7-25 micrometers of radiopaque material. A coating of radiopaque material between 7 micrometers and 15 micrometers thick helps preserve super elastic qualities of the implantable medical device.

Target material is made of a radiopaque material, for example palladium, platinum, gold, iridium, hafnium, tungsten, tantalum, rhenium, zirconium, and combinations thereof. Step 104 vaporizes the radiopaque target and deposits the radiopaque material onto the substrate implantable medical device. Vaporization and deposition can occur through any appropriate PVD process, for example those discussed with respect to FIGS. 3-4. Step 104 results in an implantable medical device with a super elastic substrate (for example substrate 92 shown in FIG. 8) and a radiopaque layer (for example radiopaque layer 94 shown in FIG. 8).

Step 106 includes vapor depositing a super elastic layer onto the radiopaque layer. In step 106, the radiopaque layer 94 and substrate 92 are acting as a substrate form for PVD. The super elastic material target is created using steps similar to steps 68-70 of method 66, as described above in relation to FIG. 6. The PVD target is vaporized and superelastic material from the target is deposited onto the radiopaque layer 94 of the substrate form during a PVD process. The super elastic material can be a nickel titanium alloy (nitinol) or any other shape-memory material. Vaporization and deposition can occur through any appropriate PVD process, for example those discussed in FIGS. 3-4. Step 106 results in implantable medical device 90 being connected to the mandrel.

Step 108 includes removing the mandrel from the PVD-processed implantable medical device 90. Removing the mandrel can be achieved through dissolving the mandrel. Any acid that attacks the material the mandrel is made of but does not attack implantable medical device 90 can be used for mandrel dissolution. Mechanical techniques can also be used to remove the mandrel. Removing the mandrel leaves a completed implantable medical device 90, as shown in FIG. 8.

For example, method 100 can be used to make implantable medical device 90 having substrate 92 and super elastic layer 96 made of a nickel titanium alloy (nitinol) and radiopaque layer 94 made of tantalum. In step 102, an implantable medical device made of a nickel titanium alloy (nitinol) is fitted onto a mandrel. In step 104, the nickel titanium alloy (nitinol) of the implantable medical device is the substrate and a tantalum target is used. During PVD in step 104, tantalum is deposited onto the nickel titanium alloy (nitinol) substrate form. The tantalum layer deposited in step 104 and the nickel titanium alloy (nitinol) substrate becomes the substrate form in step 106 and a nickel titanium alloy (nitinol) is used for the targets. Implantable medical device 90 formed during steps 102-106 is then removed from the mandrel in step 108, resulting in implantable medical device 90 with nickel titanium alloy (nitinol) and tantalum layers.

Method 100 creates implantable medical device 90 with improved radiopacity compared to an implantable medical device made only of substrate 92 (the initial implantable medical device). Coating the initial device with radiopaque material allows for better visualization of the entire body of implantable medical device 90 within a patient. Using a PVD process allows a thin coating of the radiopaque material to be applied across the entire device. Radiopaque layer 94 created by a PVD process increases the economic viability of using high-value radiopaque materials like platinum on implantable medical device 90. Using PVD is especially economical with high-value radiopaque materials compared to coring an ingot of material and drawing the ingot to the proper dimension for an implantable medical device. PVD processes require a small target made from a small ingot to form the thin coating of radiopaque material on substrate 92. Small amounts of material reduce material loss during coating of implantable medical device 90, which makes the process more economical. PVD processes like sputtering also create plasma, which cleans the substrate form to increase adhesion. PVD processes also diffuse the radiopaque material into the substrate form, creating strong diffusion bonds between the substrate form and the radiopaque layer. Thin layers of radiopaque material achieved by method 100 allows the substrate form to retain super elastic characteristics and high biocompatibility.

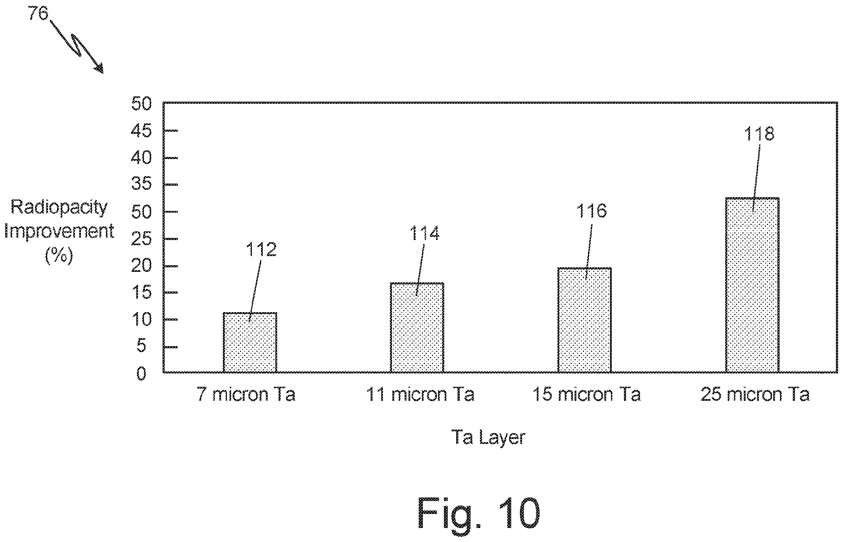

FIG. 10 is graph 110 showing predicted radiopacity improvement in implantable medical device 90. The Y-axis of graph 110 is predicted radiopacity improvement in percentage compared to an implantable medical device with no radiopaque coating. The X-axis shows various thicknesses of tantalum layers in micrometers. Tantalum layers are one example of a radiopaque layer that can be applied to a substrate, such as nitinol (e.g., a binary NiTi alloy), to form implantable medical device 90.

First bar 112 shows an approximate radiopacity improvement of 12% for a tantalum layer of 7 micrometers. Second bar 114 shows an approximate radiopacity improvement of 17% for a tantalum layer of 11 micrometers. Third bar 116 shows an approximate radiopacity improvement of 19% for a tantalum layer of 15 micrometers. Fourth bar 84 shows an approximate radiopacity improvement of 32% for a tantalum layer of 25 micrometers.

Graph 110 indicates thicker layers of tantalum between layers of a nickel titanium alloy (nitinol) in a device result in higher radiopacity improvement. Radiopacity improvement ranges from about 12% for a 7 micrometer layer of tantalum to about 32% for a 25 micrometer coating of tantalum. Increases in radiopacity improvement means a device including a layer of tantalum is more visible using X-ray techniques than a device without the layer of tantalum. Further, as the thickness of the tantalum layer increases, the radiopacity of implantable medical device 90 increases. The thickness of the tantalum layer can be selected based on the level of radiopacity needed while also ensuring implantable medical device 90 retains its super elastic characteristics.

### IMPLANTABLE MEDICAL DEVICE 120

FIG. 11 is a schematic cross section of implantable medical device 120. Implantable medical device 120 includes alloy 122.

Implantable medical device 120 as shown in FIG. 11 is a schematic representation of a cross-sectional of an implantable medical device made from alloy 122. Implantable medical device 120 can be any implantable medical device having any shape and size. Further, the cross-section shown in FIG. 11 can be a representation of a section or portion of implantable medical device 120. In the following discussion, implantable medical device 120 will be used as an example of a device that can be made from alloy 122. However, implantable medical device 120 can be any suitable medical device, including any of implantable medical devices 300, 400, 500, 600, 700, and 800 shown in FIGS. 23A-32.

Implantable medical device 120 according to the present disclosure (not part of the claimed invention) is made from alloy 122, which has radiopaque and super elastic qualities. Alloy 122 is a ternary or quaternary alloy, including a super elastic material such as a nickel titanium alloy (including nitinol) or any other shape-memory material. Alloy 122 also includes one or more radiopaque elements, including palladium, platinum, gold iridium, hafnium, tungsten, tantalum, rhenium, zirconium, and combinations thereof. Using alloy 122 for implantable medical device 120 increases the radiopacity of implantable medical device 120 compared to implantable medical devices made of a binary NiTi alloy (nitinol).

According to the presently claimed invention, alloy 122 is a ternary alloy including nickel, titanium, and platinum (NiTiPt). Platinum is added to a binary nickel-titanium alloy. Platinum composition in the alloy can range up to 44 weight percent. A first example of a NiTiPt alloy has the following atomic ratio: 42.7% nickel, 49.8% titanium, and 7.5% platinum. A second example of a NiTiPt alloy has an atomic ratio of 40.2% nickel, 49.8% titanium, and 10% platinum. A third example of a NiTiPt alloy has the following atomic ratio: 40.4% nickel, 49.6% titanium, and 10% platinum. A fourth example of a NiTiPt alloy has the following stoichiometry: Ni_{38.6}Ti_{43.4}Pt₁₈. Generally, alloys with higher atomic ratios of platinum display lower super elastic qualities. Alloys with atomic ratios of platinum higher than 10% do not exhibit super elasticity.

In an example which is not part of the claimed invention, alloy 122 is a ternary alloy including nickel, titanium, and hafnium. Atomic compositions of nickel-titanium-hafnium alloys (NiTiHf) include: Ni₅₄Ti₄₃Hf₃, Ni_{50.5}Ti_{43.5}Hf₆, Ni_{50.3}Ti_{41.7}Hf₈, Ni_{50.1}Ti_{41.8}Hf_{8.1}, and Ni₅₂Ti₃₈Hf₁₀. Ternary alloys can include hafnium from 9.3 weight percent to 26.8 weight percent. Adding hafnium to nickel and titanium creates an alloy with super elastic characteristics and improved radiopacity compared to alloys with only nickel and titanium.

In another example, alloy 122 can be an alloy with a fourth or fifth element added to the ternary alloys discussed above. Ternary alloys with a fourth element (for example, quaternary alloys) or a fifth element added have improved radiopacity while maintaining super elasticity at the service temperature of a device made with alloy 122. For implantable medical device 120, the service temperature is between 32°C and 42°C.

For example, alloy 122 can be a quaternary alloy created by adding aluminum to ternary alloys. In examples (not part of the claimed invention), aluminum can be added to alloys of nickel platinum and hafnium to create a NiTiHfAl alloy. The NiTiHfAl alloy can have a stoichiometry of Ni₅₀Ti₂₄Hf_{21.8}Al_{4.2}. In other examples useful for understanding the presently claimed invention, Aluminum can also be added to alloys of nickel, titanium, and palladium to create a NiTiPdAl alloy. The NiTiPdAl alloy can have a stoichiometry of Ni₂₀Ti₄₆Pd₃₀Al₄. In examples useful for understanding the presently claimed invention, both NiTiHfAl and NiTiPdAl can be used as alloy 122 in implantable medical device 120 for high radiopacity and maintained super elasticity.

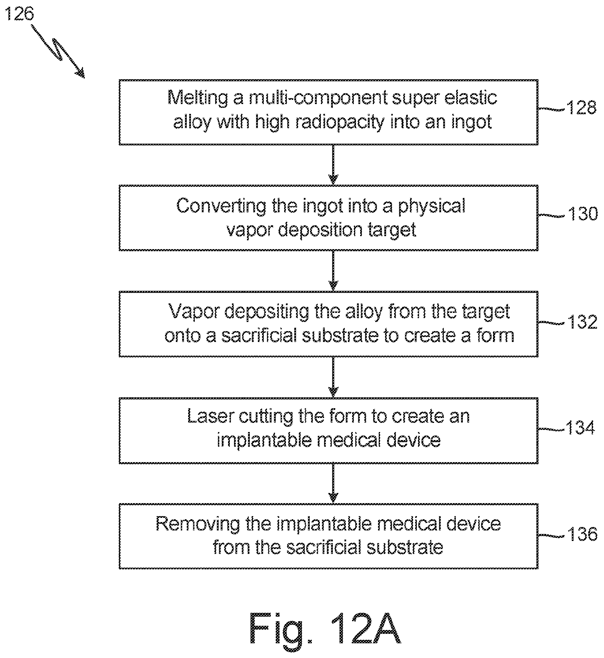

FIG. 12A is a flow chart showing method 126 of using PVD to create implantable medical device 120. Method 126 is a first example method that can be used to create implantable medical device 120 from alloy 122, as described in FIG. 11. Method 126 includes step 128, step 130, step 132, step 134, and step 136.

Method 126 can be used to make implantable medical device 120. In the following discussion, implantable medical device 120 will be used as an example of a device that can be made from method 126. However, method 126 can be used for any suitable medical device, including implantable medical devices 300, 400, 500, 600, 700, and 800 shown in FIGS. 23A-32. Method 126 can also be undergone in any suitable PVD device, including PVD devices 30 and 56 shown in FIGS. 1-2, respectively. Method 126 can also utilize any suitable PVD process, including arc-evaporation PVD (discussed with respect to FIG. 3), magnetron sputtering PVD (discussed with respect to FIG. 4), and ion beam PVD.

Step 128 includes melting a multi-component super elastic alloy with high radiopacity into an ingot. According to the present disclosure, the multi-component alloy with super elastic characteristics can be any suitable alloy, including NiTiPt, NiTiHf (not according to the claimed invention), NiTiHfAl ( not according to the claimed invention), and NiTiPdAl (not according to the claimed invention). as discussed in relation to FIG. 11. In the presently claimed invention, the multi-component alloy with super elastic characteristics is a NiTiPt alloy.

Step 130 includes converting the ingot into a physical vapor deposition target. Conversion can be accomplished by drawing, flattening, or rolling the ingot into a sheet and creating a hollow cylinder made of alloy from the sheet. The hollow cylinder can then be used as a PVD target.

Step 132 includes vapor depositing the alloy from the target onto a sacrificial substrate to create a form. The substrate is like substrate 54 in FIG. 1 or substrate 54B in FIG. 2. The substrate is a three-dimensional shape that is generally the same shape as the finished implantable medical device produced by method 126. Example shapes include tubes and cylinders. The sacrificial substrate could be a surface of a mandrel or could be attached to a mandrel. The sacrificial substrate could alternatively be a metal (for example, stainless steel) in the approximate shape of the finished device. Vapor deposition can be achieved by any reasonable vapor deposition process, for example arc-evaporation PVD (described with respect to FIG. 3), magnetron sputtering PVD (described with respect to FIG. 4), or ion beam PVD. Vapor deposition occurs until the metal alloy is between 50 micrometers and 250 micrometers thick on the sacrificial substrate. Vapor deposition can also occur until the metal alloy is between 100 micrometers and 150 micrometers thick. The resultant form is a thin layer of alloy shaped like the sacrificial substrate. For example, a cylindrical sacrificial substrate creates a tubular form. In this example, step 134 results in a thin tube made of a super elastic alloy with high radiopacity.

Step 134 includes laser cutting the form to create an implantable medical device. According to the present disclosure, the form can be laser cut into the desired shape which can include struts and openings, scored portions (not according to the claimed invention), and other apertures (not according to the claimed invention). According to the presently claimed invention, the form can be laser cut into the desired shape which includes struts and openings. Laser cutting the form creates implantable medical device 120 having the characteristics of alloy 122, as shown in FIG. 11.

Step 136 includes removing the implantable medical device from the sacrificial substrate. Removal of the sacrificial substrate can be achieved by dissolving the substrate in an acid. A proper acid for removing the substrate is an acid that attacks the material the sacrificial substrate is made from without attacking the form. Mechanical removal may also be used. The implantable medical device can have a thickness between 50 micrometers and 250 micrometers thick. More specifically, the implantable device can have a thickness between 100 micrometers and 150 micrometers thick. The implantable medical device can be a complete device or a component of an implantable medical device assembly.

Method 126 creates a relatively thin form that reduces the amount of material necessary to create a medical device. Method 126 is especially economical compared to coring an ingot of material and drawing the ingot to the proper dimension for an implantable medical device. Further, using PVD processes in method 126 is an economical process to create a medical device because less laser cutting is necessary, and less material is lost during processing. Converting the ingot to a PVD target also requires very little material and can reduce overall costs of creating a device from a ternary or quaternary alloy. Method 126 is efficient in creating medical devices because fewer laser cutting steps are necessary than a traditional wrought method. Method 126 creates a different grain structure in implantable medical device 120 compared to medical device manufactured with traditional wrought methods.

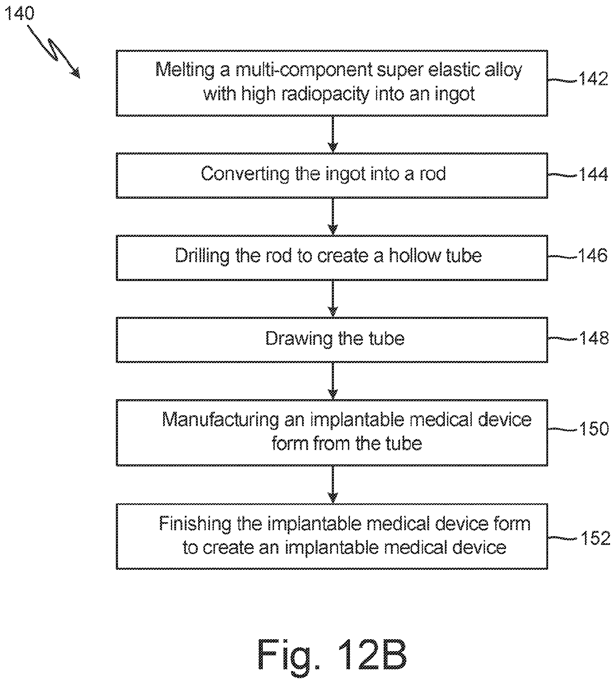

FIG. 12B is a flow chart showing method 140 of using wrought methods to create implantable medical device 120. Method 140 is a second example method that can be used to create implantable medical device 120 from alloy 122, as described in FIG. 11. Method 140 includes step 142, step 144, step 146, step 148, step 150, and step 152.

Step 142 includes melting a multi-component super elastic alloy with high radiopacity into an ingot. The super elastic alloy with high radiopacity can be any of the alloys described in FIG. 11.

Step 144 includes converting the ingot into a rod. Step 144 can be accomplished through any reasonable method.

Step 146 includes drilling the rod to create a hollow tube. Step 146 can be accomplished through any reasonable method.

Step 148 includes drawing the tube. Step 148 increases the length and decreases the width of the tube. The tube produced by step 148 should be the approximate diameter of a target implantable medical device (for example, implantable medical device 120).

Step 150 includes manufacturing an implantable medical device form from the tube. Step 150 can include laser cutting the implantable medical device form to include any structures (for example, struts and openings) necessary for the target implantable medical device. Step 150 can also include shape-setting or heat treating the implantable medical device form. Shape-setting and heat treating are processes to "train" shape memory and super-elastic materials into the form necessary for the target implantable medical device. Shape-setting and heat treating can include heating and/or cooling the implantable medical device form.

Step 152 includes finishing the implantable medical device form to create an implantable medical device. Step 152 results in a finished target implantable device. Step 152 can include finishing processes including surface finishing and sterilization. Surface finishing processes include mechanical polishing, chemical etching, chemical polishing, electropolishing, and combinations thereof. Sterilization can be performed through any reasonable sterilization technique.

Method 140 creates a different grain structure in implantable medical device 120 compared to medical device manufactured with PVD.

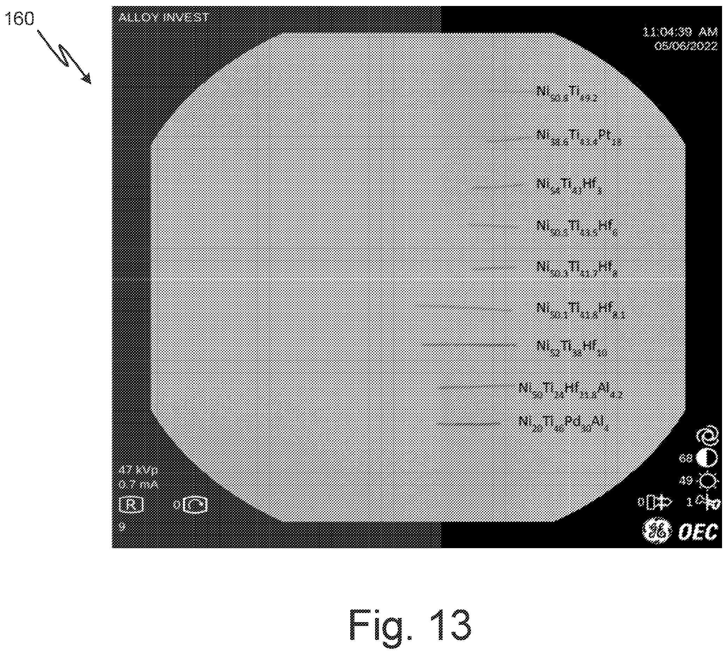

FIG. 13 is fluoroscopic image 160 comparing radiopacity of different alloy compositions. FIG. 13 shows alloys discussed in relation to FIG. 11, including NiTiPt, NiTiHf, NiTiHfAl., and NiTiPdAl. FIG. 13 includes samples of Ni_{50.8}Ti_{49.2}, Ni_{38.6}Ti_{43.4}Pt₁₈, Ni₅₄Ti₄₃Hf₃, Ni_{50.5}Ti_{43.5}Hf₆, Ni_{50.3}Ti_{41.7}Hf₈, Ni_{50.1}Ti_{41.8}Hf_{8.1}, Ni₅₂Ti₃₈Hf₁₀, Ni₅₀Ti₂₄Hf_{21.8}Al_{4.2}, and Ni₂₀Ti₄₆Pd₃₀Al₄. FIG. 13 also shows a portion of a binary nickel-titanium alloy (nitinol) under fluoroscope. Alloys with high radiopacity appear darker in fluoroscopic image 160 than alloys with lower radiopacity. FIG. 13 demonstrates how alloys ternary and quaternary alloys of nickel-titanium have higher radiopacity than a nickel-titanium alloy (nitinol) alone.

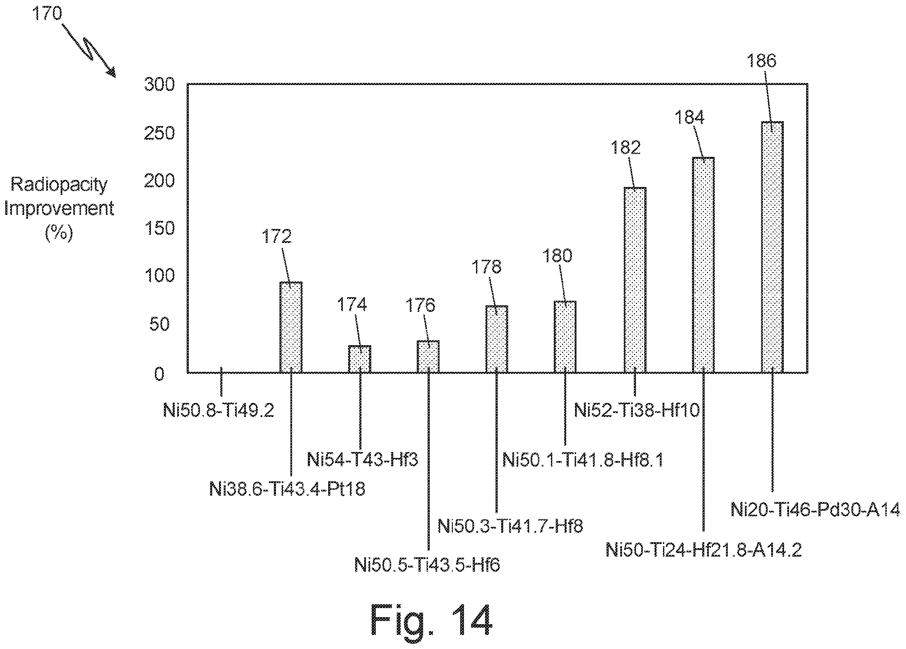

FIG. 14 is graph 170 showing predicted radiopacity improvement in implantable medical device 120. Y-axis of graph 170 is predicted radiopacity improvement in percentage compared to an implantable medical device made of a binary NiTi alloy (nitinol). X-axis shows the alloys described in relation to FIG. 11. Graph 170 includes bars 172-186.

A nickel-titanium (NiTi) alloy (nitinol) is listed on the X-axis closest to the origin of the graph. The nickel-titanium alloy has a stoichiometry of Ni_{50.8}Ti_{49.2}. No bar is included at the nickel-titanium alloy because there is an approximate 0% improvement in radiopacity. Bar 172 shows an approximately 90% radiopacity improvement for a NiTiPt alloy having a stoichiometry of Ni_{38.6}Ti_{43.4}Pt₁₈. Bar 174 shows an approximately 25% radiopacity improvement for a first NiTiHf alloy having a stoichiometry of Ni₅₄Ti₄₃Hf₃. Bar 176 shows an approximately 30% radiopacity improvement for a second NiTiHf alloy having a stoichiometry of Ni_{50.5}Ti_{43.5}Hf₆. Bar 178 shows an approximately 70% radiopacity improvement for a third NiTiHf alloy having a stoichiometry of Ni_{50.3}Ti_{41.7}Hf₈. Bar 180 shows an approximately 75% radiopacity improvement for a fourth NiTiHf alloy having a stoichiometry of Ni_{50.1}Ti_{41.8}Hf_{8.1}. Bar 182 shows an approximately 200% radiopacity improvement for fifth NiTiHf alloy having a stoichiometry Ni₅₂Ti₃₈Hf₁₀. Bar 184 shows an approximately 225% radiopacity improvement for a NiTiHfAl alloy having a stoichiometry of Ni₅₀Ti₂₄Hf_{21.8}Al_{4.2}. Bar 186 shows an approximately 260% radiopacity improvement for a NiTiPdAl alloy with a stoichiometry of Ni₂₀Ti₄₆Pd₃₀Al₄.

Graph 170 shows radiopacity improvements between 25% and 260% for different ternary or quaternary alloy shaving a super elastic alloy with a radiopaque element. Increased radiopacity results in better visualization in all portions of devices made from these alloys.

### NiTiPt ALLOYS: ALLOY 200A, ALLOY 200B, AND ALLOY 200C

Three compounds of NiTiPt (alloy 200A, alloy 200B, and alloy 200C) were tested for visualization properties, thermal properties, and mechanical properties for use in implantable medical device 60, implantable medical device 90, implantable medical device 120, as described above in relation to FIGS. 5-15, and implantable medical device 260, as described below in relation to FIGS. 20A-20D. Alloy 200A, alloy 200B, and alloy 200C can each be used as a PVD target in PVD devices 30 and 56 of FIGS. 1-2 using PVD processes, for example PVD processes described in relation to FIGS. 3A, 3B, and 4. Alloy 200A, alloy 200B, and alloy 200C can also be used to create any suitable medical device, including any of implantable medical devices 300, 400, 500, 600, 700, and 800 shown in FIGS. 23A-32.

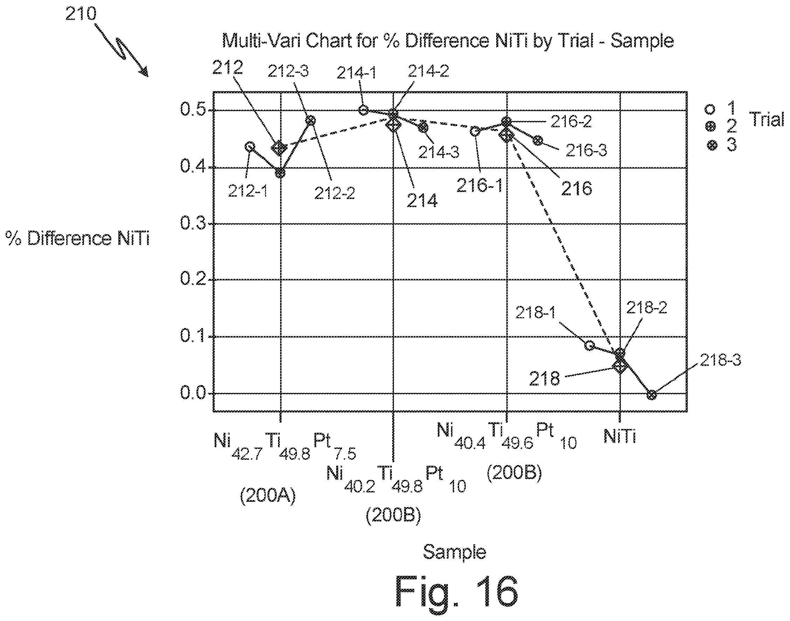

FIGS. 16-20 show results of testing alloy 200A, alloy 200B, and alloy 200C. Alloy 200A is Ni_{42.7}Ti_{49.8}Pt_{7.5}. Alloy 200B is Ni_{40.2}Ti_{49.8}Pt₁₀. Alloy 200C is Ni_{40.4}Ti_{49.6}Pt₁₀. Samples were prepared for each of alloy 200A, alloy 200B, and alloy 200C by melting and mixing each element in the ratio as listed above. Each sample was mixed six times and the finished samples were within 0.5% of the ratio listed above. Mixing each sample around twelve times will ensure each sample is consistent throughout.

FIG. 15 is fluoroscopic image 202 showing radiopacity improvement in alloy 200A, alloy 200B, and alloy 200C compared to a binary NiTi alloy (nitinol). Fluoroscopic image 202 shows samples of the binary NiTi alloy, alloy 200A, alloy 200B, and alloy 200C. Samples with high radiopacity appear darker in fluoroscopic image 202 than samples with lower radiopacity. Percent enhancement of radiopacity was calculated from fluoroscopic image 202. Alloy 200A is approximately 47% more radiopaque than the binary NiTi alloy. Alloy 200B is approximately 50% more radiopaque than the binary NiTi alloy. Alloy 200C is approximately 62% more radiopaque than the binary NiTi alloy. FIG. 15 demonstrates how alloy 200A, alloy 200B, and alloy 200C (i.e., nickel-titanium-platinum alloys) have higher radiopacity than the binary NiTi alloy (nitinol).

FIG. 16 is scatterplot 210 showing radiopacity improvement in alloy 200A, alloy 200B, and alloy 200C over a binary NiTi alloy (nitinol). Scatterplot 210 was generated from three additional trials of fluoroscopic imaging. Each result from the three trials was averaged to provide a radiopacity improvement for alloy 200A, alloy 200B, alloy 200C, and the binary NiTi alloy (control group). The average of the first trial, second trial, and third trial are shown in scatterplot 210 as a square with a plus in the center and are labeled as point 212, point 214, point 216, and point 218. Results of a first trial are shown in scatterplot 210 with an open circle and are labeled as point 212-1, point 214-1, point 216-1, and point 218-1. Results of a second trial are shown as a circle with a plus sign and are labeled as point 212-2, point 214-2, point 216-2, and point 218-2. Results of a third trial are shown as a circle with an X and are labeled as point 212-3, point 214-3, point 216-3, and point 218-3.

Scatterplot 210 shows results from testing alloy 200A (Ni_{42.2}Ti_{49.8}Pt_{7.5}) at point 212 (average), point 212-1 (test 1), point 212-2 (test 2), and point 212-3 (test 3). Alloy 200A has an approximately 40% percent difference of radiopacity improvement over the binary NiTi alloy. Results from testing alloy 200B (Ni_{40.2}Ti_{49.8}Pt₁₀) are shown at point 214 (average), point 214-1 (test 1), point 214-2 (test 2), and point 214-3 (test 3). Alloy 200B has an approximately 46% radiopacity improvement over the binary NiTi alloy. Results from testing alloy 200C (Ni_{40.4}Ti_{49.6}Pt₁₀) are shown at point 216 (average), point 216-1 (test 1), point 216-2 (test 2), and point 216-3 (test 3). Alloy 200C has an approximately 43% radiopacity improvement over the binary NiTi alloy. The binary NiTi alloy was tested as a control group. Results for testing the binary NiTi alloy are shown at point 218 (average), point 218-1 (test 1), point 218-2 (test 2), and point 218-3 (test 3). Point 218 shows an average 5% difference in radiopacity in the control group. On average, alloy 200A, alloy 200B, and alloy 200C each show an at least 30% radiopacity improvement compared to the binary NiTi alloy. As such, each of alloy 200A, alloy 200B, and alloy 200C are strong candidates for use in implantable medical devices that can be visualized during placement and follow-up care.

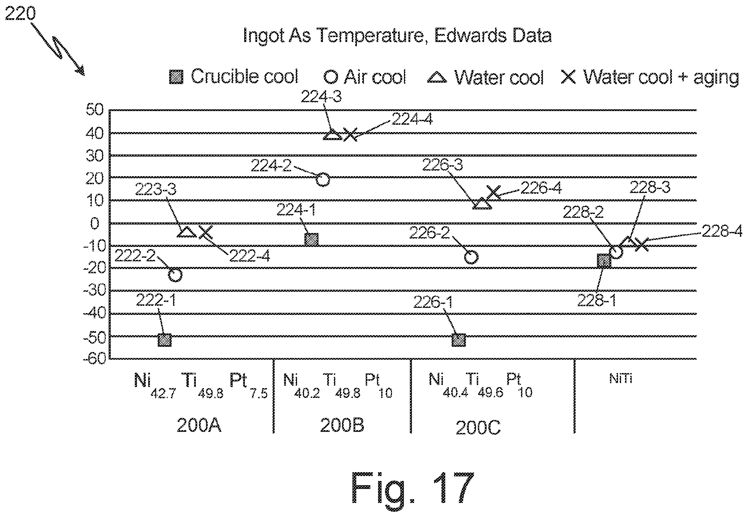

FIG. 17 is scatterplot 220 showing ingot As temperatures for ingots of alloy 200A, alloy 200B, and alloy 200C using different cooling rates. As temperature is a temperature where a metal will begin its martensitic to austenitic phase transformation; typically, material is superelastic (reform after bending) in the austenitic phase. Typical metals do not recover from deformation and instead experience plastic deformation. Implantable medical devices, such as those described in relation to FIGS. 5-15 above and in relation to FIGS. 23A-32 below should reform after bending. For example, an implantable medical device may need to bend into a catheter for delivery into a body and reform to a desired shape after deployment within the body. As such, materials used in implantable medical devices should have an As temperature below body temperature (i.e., below 37°C).

Wire specimens of alloy 200A, alloy 200B, and alloy 200C were heat treated at 830°C for 30 minutes. After heating, samples were cooled using one of four cooling methods: cooling in air in a crucible (or "crucible cooling"), cooling in air on an aluminum plate (or "plate cooling"), quenching in water (or "water cooling"), and quenching in water with an additional 30-minute low-temperature aging step (or "water cooling and aging"). The additional aging was used to reduce vacancy concentration to equilibrium levels.

Scatterplot 220 shows four different cooling methods to measure As temperature in each of alloy 200A, alloy 200B, and alloy 200C. Results from testing alloy 200A include point 222-1, point 222-2, point 222-3, and point 222-4. Results from testing alloy 200B include point 224-1, point 224-2, point 224-3, and point 224-4. Results from testing alloy 200C include point 226-1, point 226-2, point 226-3, and point 226-4. Results from testing binary nitinol as a control group include point 228-1, point 228-2, point 228-3, and point 228-4. Points indicated by squares are the results from samples which were crucible cooled. Points showing the results of crucible cooling each sample include point 222-1, point 224-1, point 226-1, and point 228-1. Points indicated by circles were plate cooled. Points showing the results of plate cooling each sample include point 222-2, point 224-2, point 226-2, and point 226-2. Points indicated by triangles were water cooled. Points showing the results of water cooling each sample include point 222-3, point 224-3, point 226-3, and point 228-3. Points indicated by X's were water cooled and aged. Points showing the results of water cooling and aging each alloy include point 222-4, point 224-4, point 226-4, and point 228-4.

Results of testing alloy 200A are shown in the leftmost column of scatterplot 220. Point 222-1 indicates alloy 200A has an approximate As temperature of -52°C after crucible cooling. Point 222-2 indicates alloy 200A has an approximate As temperature of - 23°C after plate cooling. Point 222-3 indicates alloy 200A has an approximate As temperature of -5°C after water cooling. Point 222-4 indicates alloy 200A has an approximate As temperature of around -5°C after water cooling and aging.

Results of testing alloy 200B are shown in a column second from the left of scatterplot 220. Point 224-1 indicates alloy 200B has an approximate As temperature of - 8°C after crucible cooling. Point 224-2 indicates alloy 200B has an approximate As temperature of 20°C after plate cooling. Point 224-3 indicates alloy 200B has an approximate As temperature of 40°C after water cooling. Point 224-4 indicates alloy 200B has an approximate As temperature of around 40°C after water cooling and aging.

Results of testing alloy 200C are shown in a column second from the right of scatterplot 220. Point 226-1 indicates alloy 200C has an approximate As temperature of -52°C after crucible cooling. Point 226-2 indicates alloy 200C has an approximate As temperature of -15°C after plate cooling. Point 226-3 indicates alloy 200C has an approximate As temperature of 9°C after water cooling. Point 226-4 indicates alloy 200C has an approximate As temperature of around 12°C after water cooling and aging.

Results of testing the binary NiTi alloy (nitinol) as a control are shown in the rightmost column of scatterplot 220. Point 228-1 indicates binary nitinol has an approximate As temperature of -18°C after crucible cooling. Point 226-2 indicates binary nitinol has an approximate As temperature of -12°C after plate cooling. Point 226-3 indicates binary nitinol has an approximate As temperature of -9°C after water cooling. Point 226-4 indicates binary nitinol has an approximate As temperature of around -10°C after water cooling and aging.

Results of calorimetric testing shown in scatterplot 220 indicate that cooling method of different NiTiPt alloys (i.e., alloy 200A, alloy 200B, and alloy 200C) varies the As temperature of the alloys. Scatterplot 220 also shows that aging after water cooling each of alloy 200A, alloy 200B, and alloy 200C does not significantly change As temperature of the alloys.

FIG. 18 is scatterplot 230 showing ingot As temperatures for alloy 200A and alloy 200B after heat treatment and aging at different temperatures. Scatterplot 230 shows the results for an aging study that was performed on alloy 200A and alloy 200B. Specimens of alloy 200A and alloy 200B were annealed at 830°C for 15 minutes and water quenched. After quenching, each specimen was then aged at 3 temperatures (400°C, 500°C, and 600°C) for three time intervals (10 minutes, 100 minutes, and 1,000 minutes. Scatterplot 230 shows aging time in minutes on the x-axis and As temperature in degrees Celsius on the y-axis. Points symbolized by open triangles represent specimens of alloy 200A aged at 400°C and include point 232-1, point 232-2, and point 232-3. Points symbolized by open squares represent specimens of alloy 200A aged at 500°C and include point 234-1, point 234-2, and point 234-3. Points symbolized by open circles represent specimens of alloy 200A aged at 600°C and include point 236-1, point 236-2, and point 236-3. Points symbolized by closed triangles represent specimens of alloy 200B aged at 400°C and include point 238-1, point 238-2, and point 238-3. Points symbolized by open squares represent specimens of alloy 200B aged at 500°C and include point 240-1, point 240-2, and point 240-3. Points symbolized by open circles represent specimens of alloy 200B aged at 600°C and include point 242-1 and point 242-2.

Point 232-1, point 232-2, and point 232-3 represent specimens of alloy 200A which were aged at 400°C. Point 232-1 represents a specimen of alloy 200A which was aged for 10 minutes and has an As temperature of approximately -45°C. Point 232-2 represents a specimen of alloy 200A which was aged for 100 minutes and has an As temperature of approximately -54°C. Point 232-3 represents a specimen of alloy 200A which was aged for 1000 minutes and has an As temperature of approximately -45°C.

Point 234-1, point 234-2, and point 234-3 represent specimens of alloy 200A which were aged at 500°C. Point 234-1 represents a specimen of alloy 200A which was aged for 10 minutes and has an As temperature of approximately -33°C. Point 234-2 represents a specimen of alloy 200A which was aged for 100 minutes and has an As temperature of approximately -33°C. Point 234-3 represents a specimen of alloy 200A which was aged for 1000 minutes and has an As temperature of approximately -33°C.

Point 236-1, point 236-2, and point 236-3 represent specimens of alloy 200A which were aged at 600°C. Point 236-1 represents a specimen of alloy 200A which was aged for 10 minutes and has an As temperature of approximately -21°C. Point 236-2 represents a specimen of alloy 200A which was aged for 100 minutes and has an As temperature of approximately -21°C. Point 236-3 represents a specimen of alloy 200A which was aged for 1000 minutes and has an As temperature of approximately -23°C.

Point 238-1, point 238-2, and point 238-3 represent specimens of alloy 200B which were aged at 400°C. Point 238-1 represents a specimen of alloy 200B which was aged for 10 minutes and has an As temperature of approximately -2°C. Point 238-2 represents a specimen of alloy 200B which was aged for 100 minutes and has an As temperature of approximately -3°C. Point 238-3 represents a specimen of alloy 200B which was aged for 1000 minutes and has an As temperature of approximately -3°C.

Point 240-1, point 240-2, and point 240-3 represent specimens of alloy 200B which were aged at 500°C. Point 240-1 represents a specimen of alloy 200B which was aged for 10 minutes and has an As temperature of approximately 8°C. Point 240-2 represents a specimen of alloy 200B which was aged for 100 minutes and has an As temperature of approximately 7°C. Point 240-3 represents a specimen of alloy 200B which was aged for 1000 minutes and has an As temperature of approximately 11°C.

Point 242-1 and point 242-2 represent specimens of alloy 200B which were aged at 600°C. Point 242-1 represents a specimen of alloy 200B which was aged for 10 minutes and has an As temperature of approximately 22°C. Point 242-2 represents a specimen of alloy 200B which was aged for 100 minutes and has an As temperature of approximately 24°C.

Scatterplot 230 indicates that As temperature is reduced after aging for both alloy 200A and alloy 200B. For example, As temperature of alloy 200A is approximately -5°C after water quenching *(see* FIG. 17, point 222-3). As temperature of alloy 200A is approximately -45°C after 10 minutes of aging at 400°C. Scatterplot 230 also shows that increasing aging time while keeping aging temperature and alloy composition constant does not significantly change As temperature. This indicates that if there is a precipitation reaction occurring during cooling alloy 200A or alloy 200B, the reaction is nearly complete within 10 minutes.

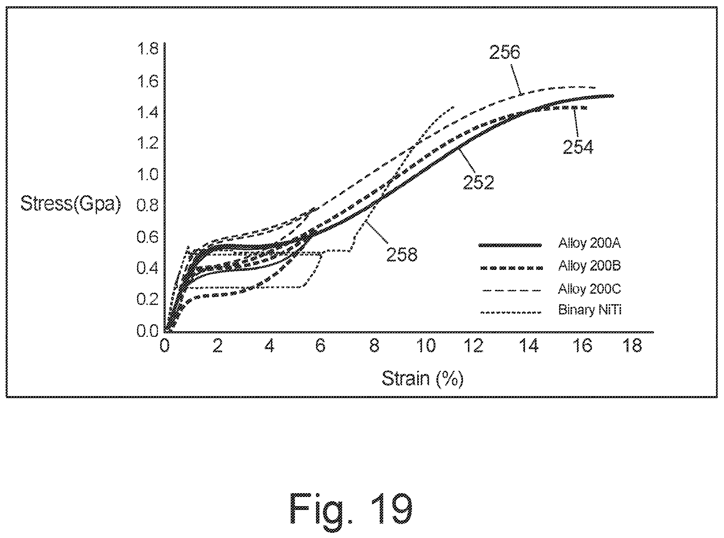

FIG. 19 shows a scatterplot of wire tensile curves. FIG. 19 shows stress in Gigapascal (GPa) on the y-axis and strain percentage on the x-axis. FIG. 19 also shows tensile curve 252, tensile curve 254, tensile curve 256, and tensile curve 258. Tensile curve 252 (solid line) represents alloy 200A. Tensile curve 254 (thick dashed line) represents alloy 200B. Tensile curve 256 (thin dashed line) represents alloy 200C. Tensile curve 258 (dotted line) represents binary nitinol. Tensile curve 258 is plotted as a control.

Wire samples of alloy 200A, alloy 200B, alloy 200C, and binary NiTi (nitinol) underwent tensile testing using a commercial testing device. Wire samples were put under stress up to approximately 1.6 Gpa. Then, the stress was released. Strain percentage was measured during stress testing.

Line 252 representing alloy 200A has a plateau like line 258 representing binary NiTi. However, the plateau in line 252 occurs at a higher stress level than the plateau in line 258. Line 254 representing alloy 200B of FIG. 19 has a sloping curve different from line 258 representing binary NiTi. However, line 254 and line 258 indicate that alloy 200B and binary NiTi have similar transformation stress because of the overlap of the transformation region, or the area having a generally quadrilateral shape. Line 254 representing alloy 200B and line 256 representing alloy 200C have a similar slope in the transformation region. Alloy 200C can withstand higher stress than alloy 200A or 200B due to the higher stress exerted compared to strain on material, as shown by line 256 compared to line 252 and line 254.

Based on the radiopacity and thermal and mechanical properties of alloy 200A, alloy 200B, and alloy 200C, the alloys can be used in implantable medical devices to help the entirety of the device be visualized during and after device placement. Alloy 200A, alloy 200B, and alloy 200C can be used as sputtering target 44 or 44B in PVD device 30 or 56, respectively, to deposit onto substrate form 54 or 54B, respectively. Alloy 200A, alloy 200B, and alloy 200C can also be incorporated into implantable medical device 60 as radiopaque coating 64 (shown in FIG. 5), implantable medical device 90 as radiopaque layer 94 (shown in FIG. 8), implantable medical device 120 as alloy 122 (shown in FIG. 11), and implantable medical device 260 as sheath 266 (shown in FIG. 15). Alloy 200A, alloy 200B, and alloy 200C is also suitable for use in medical devices including any of implantable medical devices 300, 400, 500, 600, 700, and 800 shown in FIGS. 23A-32.

### IMPLANTABLE MEDICAL DEVICE 260 (not according to the claimed invention)

FIG. 20A is a schematic cross section of implantable medical device 260. Implantable medical device 260 includes drawn filled tubing (DFT) wire 262, including core 264 and sheath 266.

Implantable medical device 260 as shown in FIG. 20A is a schematic representation of a cross-section of an implantable medical device made from DFT wire 262. Implantable medical device 260 can be any implantable medical device having any shape and size. Further, the cross-section shown in FIG. 20A can be a representation of a section or portion of implantable medical device 260. In the following discussion, implantable medical device 260 will be used as an example of a device that can be made from DFT wire 262. However, implantable medical device 260 can be any suitable medical device, including any of implantable medical devices 300, 400, 500, 600, 700, and 800 shown in FIGS. 23A-32.

DFT wire 262 includes core 264 within sheath 266. DFT wire 262 can be used to make implantable medical device 260. Core 264 is made from a material with high radiopacity including palladium, platinum, gold, iridium, hafnium, tungsten, tantalum, rhenium, zirconium, and combinations thereof. Core 264 can also be made of a nickel, titanium, and platinum alloy. Core 264 can also be made of any radiopaque alloy discussed in relation to implantable medical device 120 discussed in relation to FIGS. 11-14 above. Sheath 266 is made from a super elastic biocompatible material including a shape-memory metal. For example, sheath 266 can be made from a NiTi alloy (nitinol or binary NiTi alloy). DFT wire 262 is created by filling a rod of super elastic material with radiopaque material and then drawing the filled rod into a wire with a desired thickness. Implantable medical device 260 is made from DFT wire 262.

Implantable medical device 260 made from DFT wire 262 have improved radiopacity along at all portions formed from DFT wire 262. This helps with visualization of implantable medical device 260 within a patient's body because all portions made with DFT wire 262 can be visualized using X-ray methods. This is especially helpful in placing small devices because the entire device can be visualized. Also, DFT wire 262 with nitinol sheath 266 provides better biocompatibility.

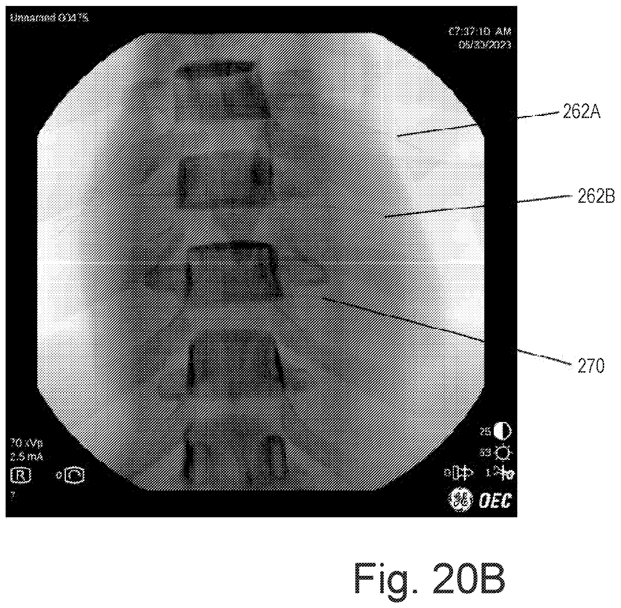

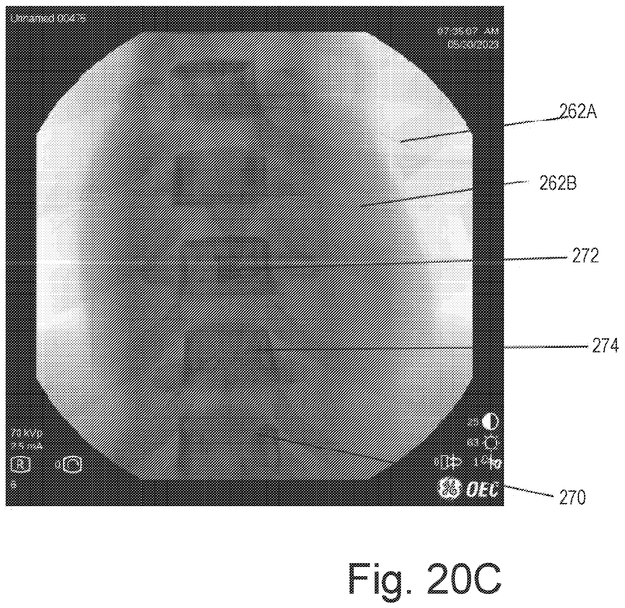

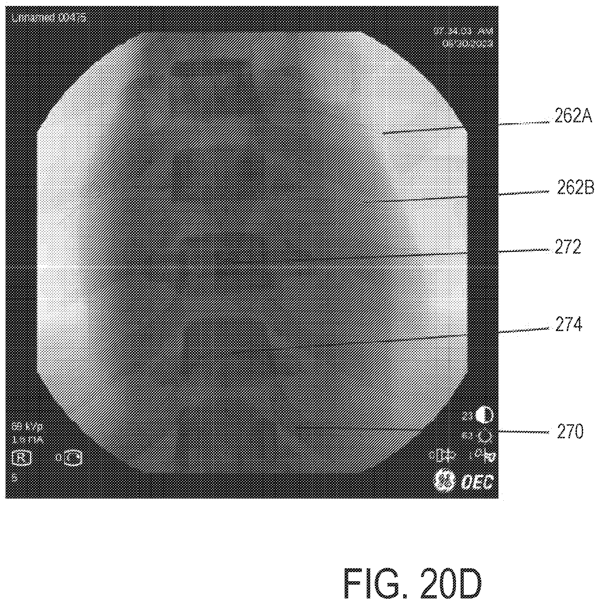

FIG. 20B is a fluoroscopic image taken of a torso model with three wire samples placed on top of the model. FIG. 20C is a fluoroscopic image taken of the torso model with three samples of wire and two sample devices placed on top. FIG. 20D is a fluoroscopic image with the three samples of wire and two sample devices placed on top. FIGS. 20B-20D will be discussed together. FIGS. 20B, 20C, and 20D show DFT wire 262A, DFT wire 262B, and binary NiTi alloy (nitinol) wire 270. FIGS. 20C, and 20D show medical device 272 and medical device 274.

FIGS. 20B-20D are fluoroscopic images taken using the same sample torso, bench, and fluoroscope. The position of the sample torso was consistent between each fluoroscopic image in FIGS. 20B-20D. The distance between the emitter and the samples was also consistent between FIGS. 20B-20D. FIGS. 20A-20B were taken with the same power settings; the emitter was set to 70 kVp and 2.5 mA. FIG. 20Dwas taken with a reduced power to reduce visibility. The power settings used to produce FIG. 20D were 69 kVp and 1.6 mA.

FIG. 20B is a fluoroscopic image of a torso model with three wire samples placed on top of the model. On vertebra 1 (near a top of FIG. 20B) is a sample of DFT wire 262A with a 10% Pt core and a binary NiTi alloy (nitinol) sheath. On vertebra 2 (just below vertebra 1) is a sample of DFT wire 262B with a 10% Ta core and a binary NiTi alloy (nitinol) sheath. On vertebra 3 (just below vertebra 2) is a sample of binary NiTi alloy (nitinol) wire 270. Each of the three wires has a gauge of 0.012 inches. As shown in FIG. 20B, DFT wire 262A and DFT wire 262B are more visible on the sample human torso than the binary NiTi alloy (nitinol) wire 270.

FIG. 20C is a fluoroscopic image of a torso model with three wire samples and two sample medical devices. On vertebra 1 (near a top of FIG. 20C) is a sample of DFT wire 262A with a 10% Pt core and a binary NiTi alloy (nitinol) sheath. On vertebra 2 (just below vertebra 1) is a sample of DFT wire 262B with a 10% Ta core and a binary NiTi alloy (nitinol) sheath. On vertebra 3 (just below vertebra 2) is a sample medical device 272 made from DFT wire 262A with a 10% Pt core and a binary NiTi alloy (nitinol) sheath. Medical device 272 is a shunt device. On vertebra 4 (below vertebra 3) is sample medical device 274 made from binary NiTi alloy (nitinol). Medical device 274 is a shunt device. On vertebra 5 (near a bottom of FIG. 20C) is a sample of binary NiTi alloy (nitinol) wire 270. Comparing medical device 272 made from DFT wire 262A and medical device 274 made from binary NiTi alloy (nitinol) in FIG. 20C, medical device 272 is more visible in FIG. 20C. Medical device 272 made from DFT wire 262A is more visible than medical device 274 made from binary NiTi alloy (nitinol).

FIG. 20D is a fluoroscopic image of a torso model with three wire samples and two sample medical devices. FIG. 20D has wire samples and sample medical devices arranged on the torso model as they were in FIG. 20C. A sample of DFT wire 262A with a 10% Pt core and a nitinol sheath is on vertebra 1; a sample of DFT wire 262B with a 10% Ta core and a nitinol sheath is on vertebra 2; a sample medical device 272 woven from DFT wire 262A is on vertebra 3; a sample medical device 274 made of nitinol is on vertebra 4; and binary NiTi alloy (nitinol) wire 270 sample is on vertebra 5. FIG. 20D is a fluoroscopic image taken with reduced power compared to FIGS. 20A-20B. Binary NiTi alloy (nitinol) wire 270 is barely visible in FIG. 20D. DFT wire 262A and DFT wire 262B on vertebra 1-2 are clearly visible even with reduced fluoroscopic power, indicating DFT wire 262A and DFT wire 262B are more radiopaque than the nitinol wire. Medical device 272 made from DFT wire 262A is also more visible on vertebra 3 than medical device 274 made of nitinol on vertebra 4, indicating devices made from DFT wire 262A having a 10% Pt core are more visible than devices made from nitinol.

### HEART H AND VASCULARTURE V

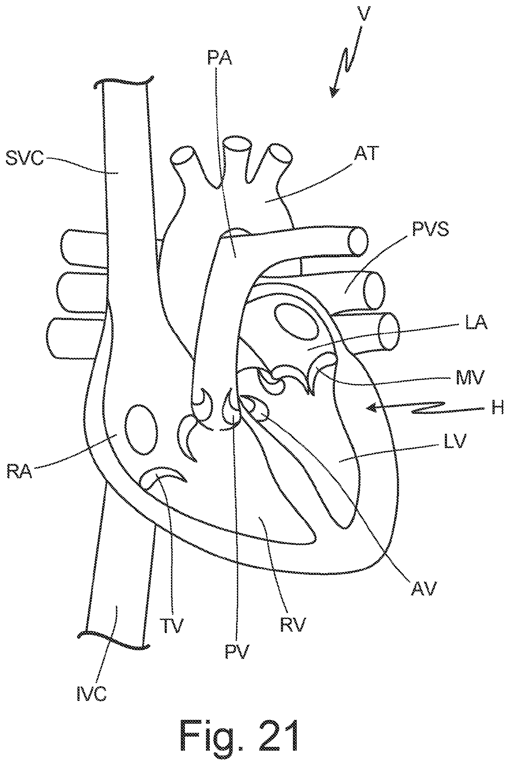

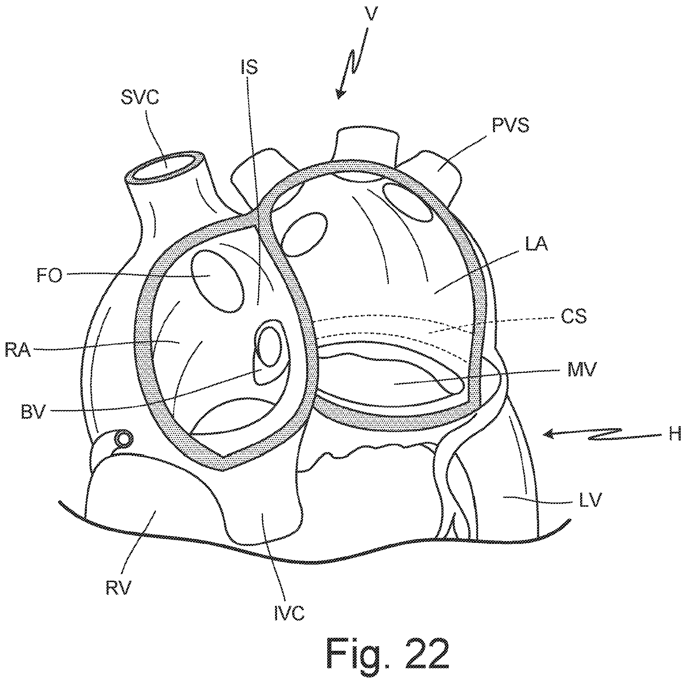

FIG. 21 is a schematic diagram of heart H and vasculature V. FIG. 22 is a cross-sectional view of heart H. FIGS. 16-17 will be discussed together. FIGS. 16-17 show heart H, vasculature V, right atrium RA, right ventricle RV, left atrium LA, left ventricle LV, superior vena cava SVC, inferior vena cava IVC, tricuspid valve TV (shown in FIG. 21), pulmonary valve PV (shown in FIG. 21), pulmonary artery PA (shown in FIG. 21), pulmonary veins PVS, mitral valve MV, aortic valve AV (shown in FIG. 21), aorta AT (shown in FIG. 21), coronary sinus CS (shown in FIG. 22), thebesian valve BV (shown in FIG. 22), inter-atrial septum IS (shown in FIG. 22), and fossa ovalis FO (shown in FIG. 22).

Heart H is a human heart that receives blood from and delivers blood to vasculature V. Heart H includes four chambers: right atrium RA, right ventricle RV, left atrium LA, and left ventricle LV.

The right side of heart H, including right atrium RA and right ventricle RV, receives deoxygenated blood from vasculature V and pumps the blood to the lungs. Blood flows into right atrium RA from superior vena cava SVC and inferior vena cava IVC. Right atrium RA pumps the blood through tricuspid valve TV into right ventricle RV. The blood is then pumped by right ventricle RV through pulmonary valve PV into pulmonary artery PA. The blood flows from pulmonary artery PA into arteries that delivery the deoxygenated blood to the lungs via the pulmonary circulatory system. The lungs can then oxygenate the blood.

The left side of heart H, including left atrium LA and left ventricle LV, receives the oxygenated blood from the lungs and pumps the blood to the body. Blood flows into left atrium LA from pulmonary veins PVS. Left atrium LA pumps the blood through mitral valve MV into left ventricle LV. The blood is then pumped by left ventricle LV through aortic valve AV into aorta AT. The blood flows from aorta AT into arteries that deliver the oxygenated blood to the body via the systemic circulatory system.

Blood is additionally received in right atrium RA from coronary sinus CS. Coronary sinus CS collects deoxygenated blood from the heart muscle and delivers it to right atrium RA. Thebesian valve BV is a semicircular fold of tissue at the opening of coronary sinus CS in right atrium RA. Coronary sinus CS is wrapped around heart H and runs in part along and beneath the floor of left atrium LA right above mitral valve MV, as shown in FIG. 22. Coronary sinus CS has an increasing diameter as it connects to right atrium RA.

Inter-atrial septum IS and fossa ovalis FS are also shown in FIG. 22. Interatrial septum IS is the wall that separates right atrium RA from left atrium LA. Fossa ovalis FS is a depression in inter-atrial septum IS in right atrium RA. At birth, a congenital structure called a foramen ovale is positioned in inter-atrial septum IS. The foramen ovale is an opening in inter-atrial septum IS that closes shortly after birth to form fossa ovalis FS. The foramen ovale serves as a functional shunt in utero, allowing blood to move from right atrium RA to left atrium LA to then be circulated through the body. This is necessary in utero, as the lungs are in a sack of fluid and do not oxygenate the blood. Rather, oxygenated blood is received from the mother. The oxygenated blood from the mother flows from the placenta into inferior vena cava IVC through the umbilical vein and the ductus venosus. The oxygenated blood moves through inferior vena cava IVC to right atrium RA. The opening of inferior vena cava IVC in right atrium RA is positioned to direct the oxygenated blood through right atrium RA and the foramen ovale into left atrium LA. Left atrium LA can then pump the oxygenated blood into left ventricle LV, which pumps the oxygenated blood to aorta AT and the systemic circulatory system. This allows the pulmonary circulatory system to be bypassed in utero. Upon birth, respiration expands the lungs, blood begins to circulate through the lungs to be oxygenated, and the foramen ovale closes to form fossa ovalis FS.

When patients have abnormal heart function, cardiovascular implants like shunts, stents, and docking devices (for example, devices 300, 400, 500, 600, 700, and 800 shown in FIGS. 18A-27) can help a patient have improved heart function. Cardiovascular implants are small and are typically made of a super elastic or shape-memory material that has a low radiopacity. The combination of small size and low radiopacity make visualizing the cardiovascular device within the heart during implantation difficult.

FIGS. 18A-27 below show different examples of cardiovascular implants that can be made with materials and methods described in FIGS. 1-15.

### SHUNT DEVICE 300

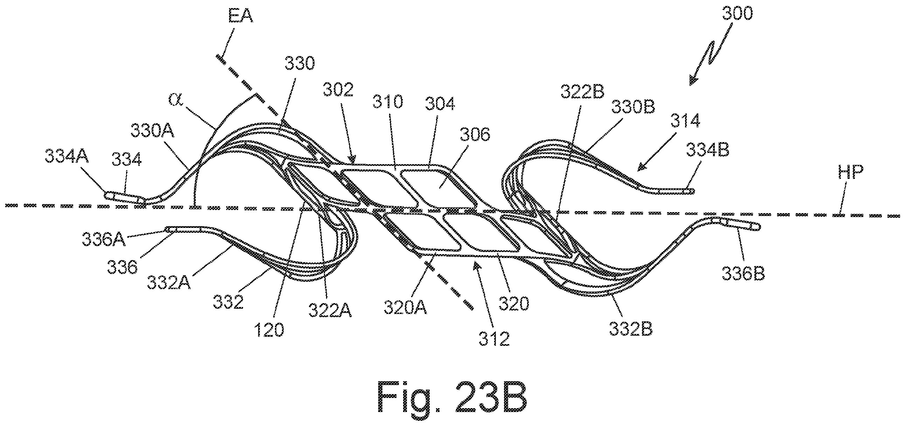

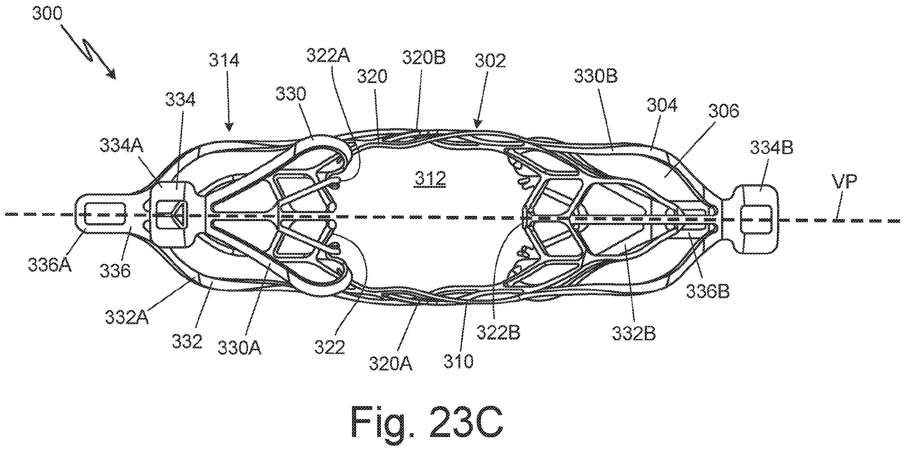

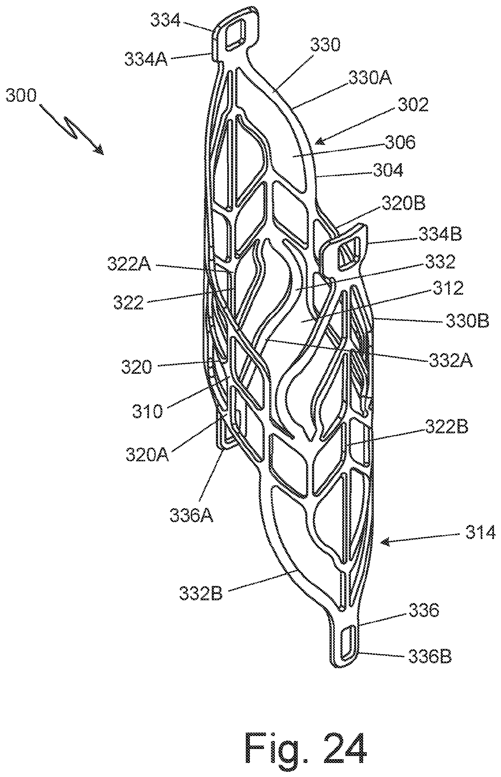

FIG. 23A is a perspective view of shunt device 300. FIG. 23B is a side view of shunt device 300. FIG. 23C is a bottom view of shunt device 300. FIG. 24 is a perspective view of shunt device 300 in a collapsed configuration. FIGS. 18A, 18B, 18C and 19 will be discussed together. Shunt device 300 includes body 302, which is formed of struts 304 and openings 306. Body 302 includes central flow tube 310, flow path 312, and arms 314. Central flow tube 310 has side walls 320 (including side wall 320A and side wall 320B) and end walls 322 (including end wall 322A and end wall 322B). Arms 314 include distal arms 330 (including distal arm 330A and distal arm 330B) and proximal arms 332 (including proximal arm 332A and proximal arm 332B). Distal arms 330 have terminal ends 334 (including terminal end 331A and terminal end 331B). Proximal arms 332 have terminal ends 336 (including terminal end 336A and terminal end 336B). FIG. 23B further shows horizontal reference plane HP, end wall axis EA, and angle α. FIG. 23C further shows vertical reference plane VP.

Shunt device 300 is shown in an expanded configuration in FIGS. 18A-18C. Shunt device 300 is formed of a super-elastic material, for example a nickel-titanium alloy (nitinol), that is capable of being compressed into a catheter for delivery into the body. Shunt device 300 is shown in a compressed configuration in FIG. 24. Upon delivery into the body, shunt device 300 will expand back to its relaxed, or expanded, shape. Shunt device 300 has body 302 that is formed of interconnected struts 304. Openings 306 in body 302 are defined by struts 304. Body 302 of shunt device 300 is formed of struts 304 to increase the flexibility of shunt device 300 to enable it to be compressed and expanded. Shunt device 300 can be sterilized before being delivered into the body.

Body 302 includes central flow tube 310 that forms a center portion of shunt device 300. Central flow tube 310 is tubular in cross-section, but is formed of struts 304 and openings 306. Central flow tube 310 can be positioned in a puncture in a tissue wall and holds the tissue wall open. Flow path 312 is an opening extending through central flow tube 310. Flow path 312 is the path through which blood flows through shunt device 300. Arms 314 extend from central flow tube 310. Arms 314 extend outward from central flow tube 310 when shunt device 300 is in an expanded configuration. Arms 314 hold shunt device 300 in position in the tissue wall when shunt device 300 is implanted in the body.

When shunt device 300 is implanted in the tissue wall between the left atrium and the coronary sinus, central flow tube 310 holds the tissue wall open so blood can flow from the left atrium to the coronary sinus through flow path 312. Struts 304 of central flow tube 310 form a cage of sorts that is sufficient to hold the tissue wall open around central flow tube 310. Central flow tube 310 is designed to have a thickness that approximates the thickness of the tissue wall between the left atrium and the coronary sinus.

Central flow tube 310 has side walls 320 and end walls 322. Side wall 320A and side wall 320B form opposing sides of central flow tube 310. End wall 322A and end wall 322B form opposing ends of central flow tube 310. End wall 322A and end wall 322B each extend between and connect to side wall 320A and side wall 320B to form a circular opening that defines flow path 312. Struts 304 of central flow tube 310 define generally parallelogram-shaped openings 306 in central flow tube 310. Struts 304 of side walls 320 form an array of parallelogram-shaped openings 306 in side walls 320. Side walls 320 and end walls 322 form a tubular lattice for central flow tube 310.

As shown in FIG. 23B, central flow tube 310 is angled with respect to horizontal reference plane HP extending through shunt device 300. Horizontal reference plane HP lies generally in the plane of the tissue wall immediately adjacent to shunt device 300 when shunt device 300 is implanted. End walls 322 are angled with respect to horizontal reference plane HP. As shown in FIG. 23B, end walls 322 extend along end wall axis EA that extends at angle α with respect to horizontal reference plane HP. Angle α can be between 15° and 90°. Alternatively, angle α can be between 30° and 75°. Alternatively, angle α can be between 60° and 65°.

Arms 314 of shunt device 300 include two distal arms 330 and two proximal arms 332. Arms 314 extend outward from end walls 322 of central flow tube 310 when shunt device 300 is in an expanded configuration. Distal arm 330A is connected to and extends away from end wall 322A, and distal arm 330B is connected to and extends away from end wall 322B. Proximal arm 332A is connected to and extends away from end wall 322A, and proximal arm 332B is connected to and extends away from end wall 322B. When shunt device 300 is implanted in the tissue wall between the left atrium and the coronary sinus, distal arms 330 will be positioned in the left atrium and proximal arms 332 will be positioned in the coronary sinus.

Distal arms 330 and proximal arms 332 curl outward from end walls 322. As shown in FIG. 23C, distal arm 330A and distal arm 330B extend outwards from central flow tube 310 in opposite directions parallel to vertical reference plane VP. Distal arm 330A has a longer length than distal arm 330B. Proximal arm 332A and proximal arm 332B extend outwards from central flow tube 310 in opposite directions parallel to vertical reference plane VP. Proximal arm 332A has a shorter length than proximal arm 332B. Distal arm 330A has generally the same length and shape as proximal arm 332B, and distal arm 330B has generally the same length and shape as proximal arm 332A. As such, shunt device 300 is inversely symmetrical across horizontal reference plane HP, as shown in FIG. 23B.

Shunt device 300 is generally elongated longitudinally but is relatively narrow laterally. Stated another way, distal arms 330 and proximal arms 332 are not annular or circular, but rather extend outward generally in only one plane. As shown in FIG. 23B, shunt device 300 has a generally H-shape when viewing a side of shunt device 300. The elongated shape of shunt device 300 means that when compressed it elongates along a line, as shown in FIG. 24, so as to better fit within a catheter.

Distal arms 330 each have terminal ends 334. Specifically, distal arm 330A has terminal end 331A, and distal end 330B has terminal end 331B. Proximal arms 332 each have terminal ends 336. Specifically, proximal arm 332A has terminal end 336A, and proximal arm 332B has terminal end 336B. Terminal ends 334 of distal arms 330 and terminal ends 336 of proximal arms 332 converge towards one another. Distal arms 330 and proximal arms 332 form two pairs of arms. Distal arm 330A and proximal arm 332A form a first pair of arms extending outward from a first side of central flow tube 310, and terminal end 339A of distal arm 330A converges towards terminal end 136A of proximal arm 332A. Distal arm 330B and proximal arm 332B form a second pair of arms extending outward from a second side of central flow tube 310, and terminal end 339B of distal arm 330B converges towards terminal end 336B of proximal arm 332B. The gap between terminal ends 334 and terminal ends 336 is sized to be slightly smaller than an approximate thickness of the tissue wall between the left atrium and the coronary sinus. This allows distal arms 330 and proximal arms 332 to flex outwards and grip the tissue wall when implanted to help hold shunt device 300 in place in the tissue wall. Terminal ends 334 of distal arms 330 and terminal ends 336 of proximal arms 332 can also have openings that are configured to engage a delivery tool to facilitate implantation of shunt device 300, for example actuating rods of a delivery tool.

When implanted in the tissue wall, distal arms 330 and proximal arms 332 are designed such that the projection of distal arms 330 and proximal arms 332 into the left atrium and the coronary sinus, respectively, is minimized. This minimizes the disruption of the natural flow patterns in the left atrium and the coronary sinus. Shunt device 300 can also be designed so that the profile of proximal arms 332 projecting into the coronary sinus is lower than the profile of distal arms 330 projecting into the left atrium to minimize disruption of the natural blood flow through the coronary sinus.

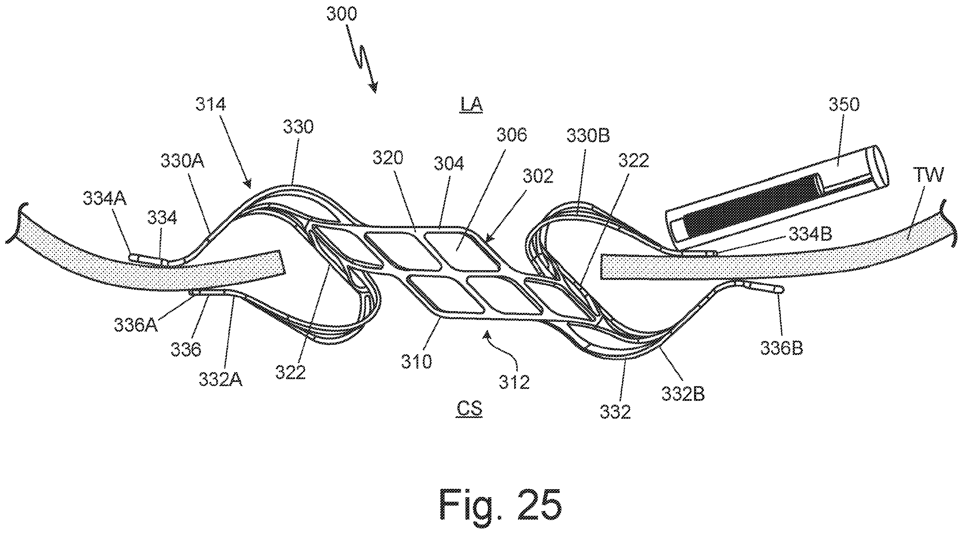

FIG. 25 is a side view of shunt device 300 with sensor 350 and anchored to tissue wall TW. Shunt device 300 includes body 302, which is formed of struts 304 and openings 306. Body 302 includes central flow tube 310, flow path 312, and arms 314. Central flow tube 310 has side walls 320 and end walls 322. Arms 314 include distal arms 330 (including distal arm 330A and distal arm 330B) and proximal arms 332 (including proximal arm 332A and proximal arm 332B). Distal arms 330 have terminal ends 334 (including terminal end 331A and terminal end 331B). Proximal arms 332 have terminal ends 336 (including terminal end 336A and terminal end 336B). FIG. 25 further shows sensor 350, tissue wall TW, left atrium LA, and coronary sinus CS.

Shunt device 300 is described above in reference to FIGS. 18A-20. Shunt device 300 as shown in FIG. 25 further includes sensor 350. Shunt device 300 is shown implanted in tissue wall TW. In the example shown in FIG. 25, sensor 350 will be positioned in left atrium LA when shunt device 300 is implanted in tissue wall TW. Sensor 350 is attached to distal arm 330B of shunt device 300 in the example shown in FIG. 25, but can be attached to distal arm 330A in alternate examples. In further examples, sensor 350 can be attached to proximal arm 332A or proximal arm 332B to be positioned in coronary sinus CS. Alternatively, an additional sensor can be included on shunt device 300 to position a sensor in both left atrium LA and coronary sinus CS. Sensor 350 can be integrally formed with shunt device 300 or attached to shunt device 300 using any suitable mechanism.

Sensor 350 can be a pressure sensor to sense a pressure in the left atrium. In other examples, sensor 350 can be any sensor to measure a parameter in the left atrium. Sensor 350 can include a transducer, control circuitry, and an antenna in one example. The transducer, for example a pressure transducer, is configured to sense a signal from the left atrium. The transducer can communicate the signal to the control circuitry. The control circuitry can process the signal from the transducer or communicate the signal from the transducer to a remote device outside of the body using the antenna. Sensor 350 can include alternate or additional components in other examples. Further, the components of sensor 350 can be held in a sensor housing that is hermetically sealed.

Shunt device 300 can be manufactured at least in part using a PVD process as described above in reference to FIGS. 1-14 to improve the radiopacity of shunt device 300. For example, shunt device 300 can have a radiopaque coating applied to body 302, similar to implantable medical device 60 as shown in and discussed in reference to FIGS. 5-7. Shunt device 300 can also have a radiopaque layer and a super elastic layer applied to body 302, similar to implantable medical device 90 as shown in and discussed in reference to FIGS. 8-10. Body 302 of shunt device 300 can also be manufactured out a ternary or quaternary metal alloy having radiopaque and super elastic properties, similar to implantable medical device 120 as shown in and discussed in reference to FIGS. 11-14. Alternatively, body 302 of shunt device 300 can be manufactured using a DFT wire having a radiopaque core and a super elastic sheath, similar to implantable medical device 260 shown in and discussed in reference to FIG. 15.

### CARDIOVASCULAR IMPLANT DEVICE 400 (not according to the claimed invention)

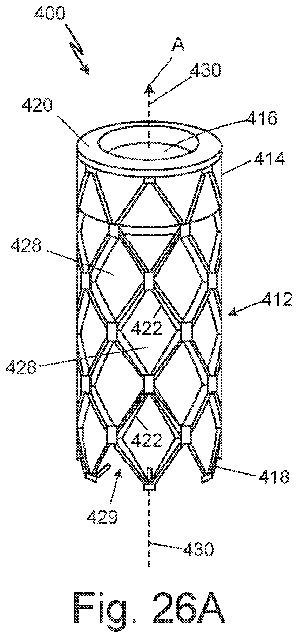

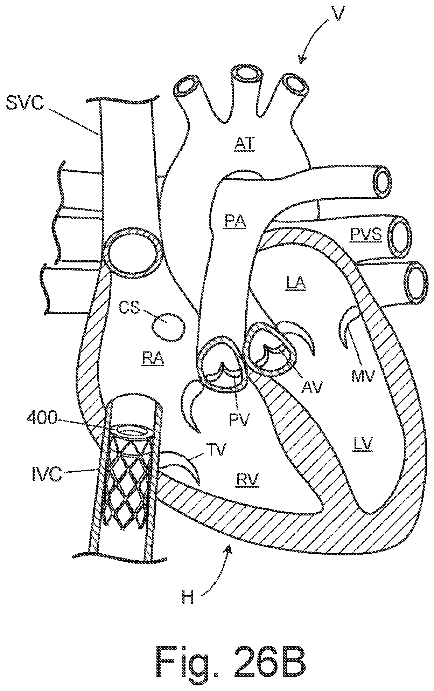

FIG. 26A is a perspective view of cardiovascular implant device 400. FIG. 26B is a sectional view of heart H illustrating an example positioning at a non-valve site of cardiovascular implant device 400.

As illustrated in FIG. 26A, cardiovascular implant device 400 includes frame 412, cover 414, valve seat 416, inflow end 418, and outflow end 420. Frame 412 includes struts 422, inner diameter 424, inner surface 425, outer diameter 426, and outer surface 427, and defines openings 428, central flow path 429, and flow axis 430. FIG. 26B also shows device 400, heart H, vasculature V, right atrium RA, right ventricle RV, left atrium LA, left ventricle LV, superior vena cava SVC, inferior vena cava IVC, tricuspid valve TV, pulmonary valve PV, pulmonary artery PA, pulmonary veins PVS, mitral valve MV, aortic valve AV, aorta AT, and coronary sinus CS.

Cardiovascular implant device 400 is an implantable device for use in a cardiovascular system. Cardiovascular implant device 400 is configured to be implanted in blood vessels or chambers of heart H. In the illustrated example, cardiovascular implant device 400 is a "40resent" or docking station for supporting a valve device, such as a prosthetic valve device. Cardiovascular implant device 400 can be delivered into the cardiovascular system via a catheter (i.e., transcatheter delivery) or can be surgically placed using transcatheter or surgical procedures known in the art. As shown in FIG. 26B, device 400 is located in inferior vena cava IVC near its opening into right atrium RA. That is, device 400 is located at a site in heart H where there is not naturally a valve (a "non-valve" site). In other examples, device 400 can be located in superior vena cava SVC. In yet other examples, device 400 can be located in any vessel or chamber of heart H at a non-valve site or at a site where there is a natural valve (e.g., aortic valve AV, mitral valve MV, pulmonary valve PV, etc.). Examples of a cardiovascular implant device in a valve site are described below with reference to FIGS. 22A-24.

Frame 412 forms a main body of device 400. Frame 412 can be expandable. Frame 412 can have a wide variety of different shapes and sizes. As shown in FIGS. 21A-21B, frame 412 is an annular or cylindrical mesh or lattice. Frame 412 has inner diameter 424 and outer diameter 426. Each of inner diameter 424 and outer diameter 426 can vary along a length of frame 412. Inner diameter 424 is the diameter of radially inner surface 425 of frame 412. Outer diameter 426 is the diameter of radially outer surface 427 of frame 412. Frame 412 can have any suitable length. For example, frame 412 may be approximately as long as a valve that is configured to sit within frame 412 (e.g., within valve seat 416). In other examples, frame 412 can be longer or shorter than a valve that is configured to sit within frame 412. Frame 412 can press against or into tissue walls at the implant site or contour (or extend) around anatomical structures of the cardiovascular system to set and maintain the position of device 400.

Frame 412 can be formed in a variety of ways, e.g., connecting individual wires together to form a mesh or lattice, braiding, cutting from a sheet and then rolling or otherwise forming into the shape of frame 412, molding, cutting from a cylindrical tube (e.g., cutting from a nitinol tube), other ways, or a combination of these. Frame 412 can be made from a highly flexible metal, metal alloy, or polymer. Examples of metals and metal alloys that can be used include, but are not limited to, nitinol and other shape-memory alloys, non-magnetic cobalt-chromium-nickel-molybdenum alloys (Elgiloy), and stainless steel, but other metals and highly resilient or compliant non-metal materials can be used to make frame 412. All or a portion of frame 412 can be monolithically formed of any of these materials. These materials can allow frame 412 to be compressed to a small size, and then-when the compression force is released-frame 412 can self-expand back to its pre-compressed shape. Frame 412 can expand back to its pre-compressed shape due to the material properties frame 412 is made of and/or frame 412 can be expanded by inflation or expansion of a device positioned inside frame 412. For example, frame 412 can be compressed such that frame 412 can fit into a delivery catheter. Frame 412 can also be made of other materials and can be expandable and collapsible in different ways, e.g., mechanically-expandable, balloon-expandable, self-expandable, or a combination of these.

Frame 412 extends between inflow end 418 and outflow end 420 of cardiovascular implant device 400. Inflow end 418 can be an end of device 400 that is relatively upstream of outflow end 420 with respect to a flow of blood along flow axis 430, as represented by arrow A in FIG. 26A, when device 400 is implanted in a blood vessel or chamber of heart H. Accordingly, outflow end 420 is an end of device 400 that is relatively downstream of inflow end 418 with respect to a flow of blood along flow axis 430, as represented by arrow A in FIG. 26A, when device 400 is implanted in a blood vessel or chamber of heart H. In the example shown in FIG. 26B, outflow end 420 is positioned near where inferior vena cava IVC opens into right atrium RA and inflow end 418 is positioned upstream within inferior vena cava IVC. Although inflow end 418 is defined as being relatively upstream of outflow end 420, it should be understood that other actual positions of inflow end 418 or outflow end 420 are possible depending on the location where device 400 is implanted.

Frame 412 is formed of a plurality of struts 422. Struts 422 make up the lattice or mesh of frame 412 and define openings (or cells) 428 therein. Struts 422 can be integrally formed. In some examples, all or a portion of struts 422 are monolithically formed from the same material. Openings 428 extend through frame 412 from inner surface 425 to outer surface 427. Each of openings 428 is bounded on one or more sides by ones of struts 422. Openings 428 can have any suitable shape or size, which can in turn be based on an overall shape or size of frame 412. In the example shown in FIG. 26A, openings 428 are diamond shaped and arranged in circumferential rows around frame 412. In other examples, openings 428 can have any other regular or irregular polygonal or non-polygonal shape and pattern. In some examples, ones of openings 428 can have different shapes or sizes throughout frame 412. In some examples, ones of openings 428 can be connected by gaps to adjacent ones of openings 428.

Central flow path 429 is an open channel through a central portion of annular frame 412. Central flow path 429 is defined by inner surface 425 of frame 412. Central flow path 429 extends from inflow end 418 to outflow end 420 such that device 400 is open at each end. Accordingly, blood flowing through and out of device 400 follows central flow path 429. More specifically, flow axis 430 is a longitudinal axis through device 400 along which blood flows as it passes or is directed through device 400 (e.g., in the direction indicated by arrow A in FIG. 26A). In the example illustrated in FIG. 26B where device 400 is implanted in inferior vena cava IVC, a valve seated in device 400 can open when heart H is in a diastolic phase. Blood flows from inferior vena cava IVC and superior vena cava SVC into right atrium RA. The blood that flows from inferior vena cava IVC flows through device 400 along flow axis 430. During the diastolic phase, blood in right atrium RA flows through tricuspid valve TV and into right ventricle RV. During a systolic phase of heart H, a valve seated in device 400 can close. Blood is prevented from flowing (i.e., backflowing) from right atrium RA into inferior vena cava IVC by the closed valve in device 400. A closed valve in device 400 prevents any blood that regurgitates through the through tricuspid valve TV during the systolic phase from being forced into inferior vena cava IVC.

Cover 414 is a covering for one or more portions of frame 412. Cover 414 can be a fabric material, a polymer material, or other material. For example, cover 414 can be a material that promotes tissue ingrowth where device 400 contacts adjacent tissue walls of a vessel or chamber of heart H. Cover 414 can also form a seal to limit or prevent blood flow through portions of frame 412 that are covered by cover 414. Cover 414 can be attached to frame 412 by any suitable attachment means, such as by stitching, gluing, tying, etc. Cover 414 can be shaped and positioned in a variety of ways. In the example shown in FIG. 26A, cover 414 is adjacent to outflow end 420. In some examples, cover 414 is near or adjacent valve seat 416. In other examples, cover 414 can be adjacent to inflow end 418 or at any location or locations between inflow end 418 and outflow end 420. In yet other examples, device 400 does not include cover 414.

Valve seat 416 is a portion of device 400 for holding, supporting, or attaching to a valve device, such as a prosthetic valve device. In some examples, valve seat 416 can be a portion of frame 412. In some examples, valve seat 416 can be monolithically formed with frame 412. In other examples, valve seat 416 can be formed separately from frame 412 and attached. Valve seat 416 can take any form that provides a supporting surface for implanting or deploying a valve within device 400 after device 400 is implanted in the cardiovascular system. In the example shown in FIG. 26A, valve seat 416 is located near outflow end 420. However, it should be understood that in other examples valve seat 416 can be located at any lengthwise position along frame 412. Valve seat 416 (and a valve seated therein, not shown) can span across a portion of central flow path 429. Valve seat 416 allows a valve to be implanted in vasculature or tissue of varying strengths, sizes, and shapes. The outer profile of device 400 (e.g., outer surface 427 of frame 412) can better conform to the cardiovascular anatomy (e.g., vasculature, tissue, heart, etc.) without putting too much pressure on the anatomy, while a valve can be firmly and securely implanted in valve seat 416 to prevent or mitigate the risk of migration or slipping.

Once device 400 is implanted in cardiovascular system (e.g., in inferior vena cava IVC as shown in FIG. 26B), circulating blood passes through device 100.

Cardiovascular implant device 400 can be manufactured at least in part using a PVD process as described above in reference to FIGS. 1-14 to improve the radiopacity of cardiovascular implant device 400. For example, cardiovascular implant device 400 can have a radiopaque coating applied to frame 412, similar to implantable medical device 60 as shown in and discussed in reference to FIGS. 5-7. Cardiovascular implant device 400 can also have a radiopaque layer and a super elastic layer applied to frame 412, similar to implantable medical device 90 as shown in and discussed in reference to FIGS. 8-10. Frame 412 of cardiovascular implant device 400 can also be manufactured out a ternary or quaternary metal alloy having radiopaque and super elastic properties, similar to implantable medical device 120 as shown in and discussed in reference to FIGS. 11-14. Alternatively, frame 412 of cardiovascular implant device 400 can be manufactured using a DFT wire having a radiopaque core and a super elastic sheath, similar to implantable medical device 260 shown in and discussed in reference to FIG. 15.

### CARDIOVASCULAR IMPLANT DEVICE 500 (not according to the claimed invention)

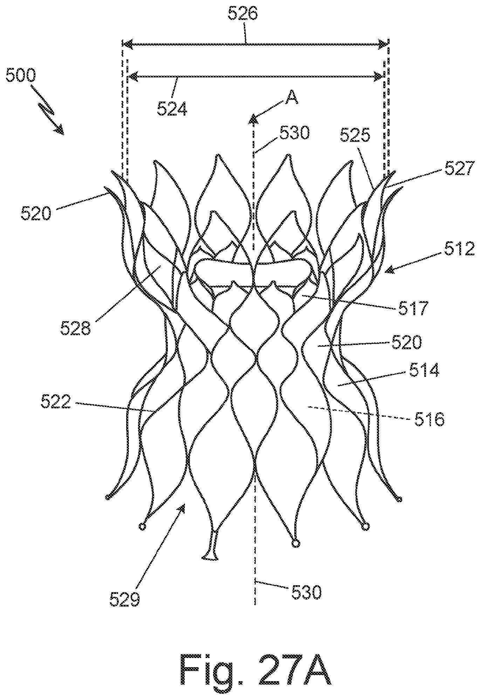

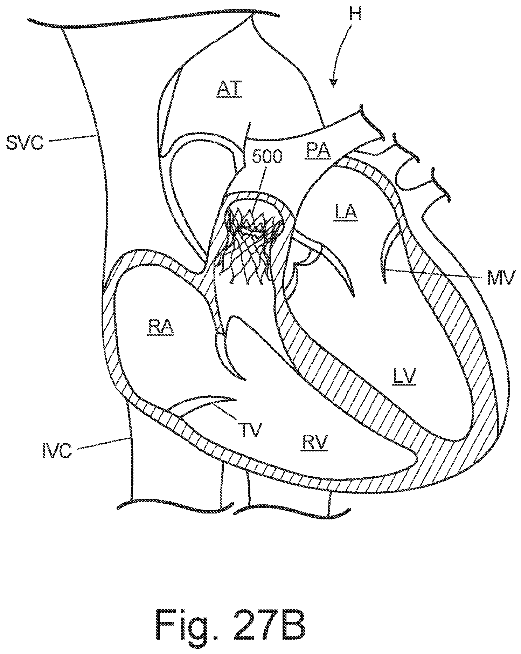

FIG. 27A is a perspective view of cardiovascular implant device 500. FIG. 27B is a sectional view of heart H illustrating an example positioning at a valve site of cardiovascular implant device 500. FIGS. 22A-22B will be described together. As illustrated in FIGS. 22A-22B, cardiovascular implant device 500 includes frame 512, cover 514, valve seat 516, valve 517, inflow end 518, and outflow end 520. Frame 512 includes struts 522, inner diameter 524, inner surface 525, outer diameter 526, and outer surface 527, and defines openings 528, central flow path 529, and flow axis 530. FIG. 27B also shows device 500, heart H, right atrium RA, right ventricle RV, left atrium LA, left ventricle LV, superior vena cava SVC, inferior vena cava IVC, tricuspid valve TV, pulmonary valve PV, pulmonary artery PA, mitral valve MV, aortic valve AV, and aorta AT.

Cardiovascular implant device 500 includes a similar structure and function to cardiovascular implant device 400 described above, except device 500 is located at a valve site rather than a non-valve site. For example, FIG. 27B shows device 500 is positioned in pulmonary artery PA at the site of pulmonary valve PV such that inflow end 518 is facing right ventricle RV and outflow end 520 is within pulmonary artery PA. In other examples, device 500 can be located at aortic valve AV, mitral valve MV, or other valves. Cardiovascular implant device 500 also includes various minor structural variations compared to device 400. For example, frame 512 has a bi-directionally flared or generally hourglass shaped profile rather than a regular cylindrical shape. Valve seat 516 is located centrally along a longitudinal axis (e.g., flow axis 530) through cardiovascular implant device 500. Cardiovascular implant device 500 is also depicted in FIGS. 22A-22B as including valve 517 supported in valve seat 516. Compared to cover 414 for device 400 as shown in FIG. 26A, cover 514 extends over a greater portion of frame 512. Moreover, cover 514 extends from inflow end 518 towards outflow end 520 but outflow end 520 is not covered by cover 514. It should be understood that, among other variations described above with reference to device 400, other examples of cardiovascular implant devices can include a wide variety of frame shapes and sizes and positions of valve seats and covers.

Once device 500 is implanted in cardiovascular system (e.g., in pulmonary artery PA as shown in FIG. 21), circulating blood passes through device 500.

Cardiovascular implant device 500 can be manufactured at least in part using a PVD process as described above in reference to FIGS. 1-14 to improve the radiopacity of cardiovascular implant device 500. For example, cardiovascular implant device 500 can have a radiopaque coating applied to frame 512, similar to implantable medical device 60 as shown in and discussed in reference to FIGS. 5-7. Cardiovascular implant device 500 can also have a radiopaque layer and a super elastic layer applied to frame 512, similar to implantable medical device 90 as shown in and discussed in reference to FIGS. 8-10. Frame 512 of cardiovascular implant device 500 can also be manufactured out a ternary or quaternary metal alloy having radiopaque and super elastic properties, similar to implantable medical device 120 as shown in and discussed in reference to FIGS. 11-14. Alternatively, frame 512 of cardiovascular implant device 500 can be manufactured using a DFT wire having a radiopaque core and a super elastic sheath, similar to implantable medical device 260 shown in and discussed in reference to FIG. 15.

### CARDIOVASCULAR IMPLANT DEVICE 600 (not according to the claimed invention)

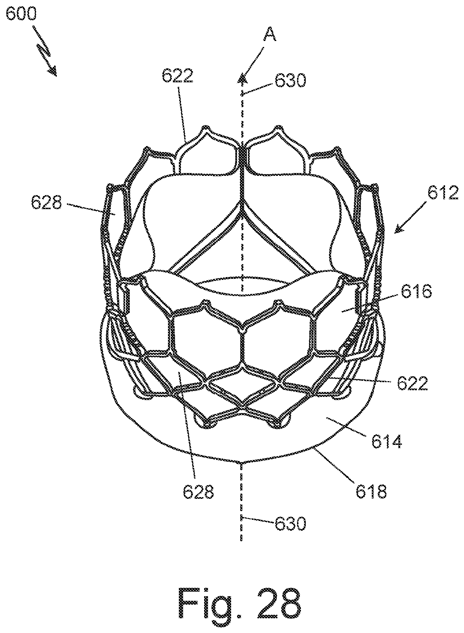

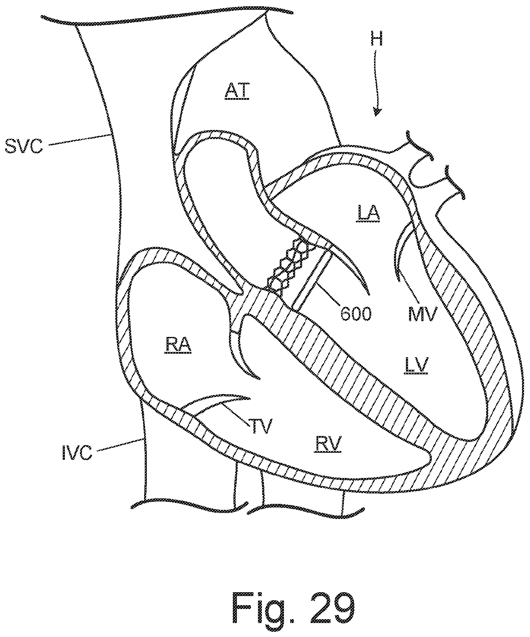

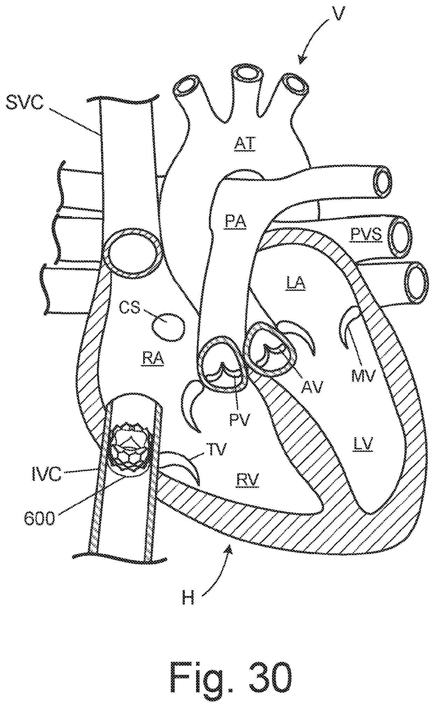

FIG. 28 is a perspective view of cardiovascular implant device 600. FIG. 29 is a sectional view of heart H illustrating an example positioning at a valve site of cardiovascular implant device 600. FIG. 30 is a sectional view of heart H illustrating an example positioning at a non-valve site of cardiovascular implant device 600. FIGS. 23-25 will be described together.

As illustrated in FIG. 28, cardiovascular implant device 600 includes frame 612, cover 614, valvular body 616, inflow end 618, and outflow end 620. Frame 612 includes struts 622, inner diameter 624, inner surface 625, outer diameter 626, and outer surface 627, and defines openings 628, central flow path 629, and flow axis 630. Valvular body includes leaflets 631. FIG. 29 also shows device 600, heart H, right atrium RA, right ventricle RV, left atrium LA, left ventricle LV, superior vena cava SVC, inferior vena cava IVC, tricuspid valve TV, mitral valve MV, aortic valve AV, and aorta AT. FIG. 30 also shows device 600, heart H, vasculature V, right atrium RA, right ventricle RV, left atrium LA, left ventricle LV, superior vena cava SVC, inferior vena cava IVC, tricuspid valve TV, pulmonary valve PV, pulmonary artery PA, pulmonary veins PVS, mitral valve MV, aortic valve AV, aorta AT, and coronary sinus CS.

Cardiovascular implant device 600 is an implantable device for use in a cardiovascular system. Cardiovascular implant device 600 is configured to be implanted in blood vessels or chambers of heart H. In the illustrated example, cardiovascular implant device 600 is a valve device, such as a prosthetic valve device. In some examples, device 600 is deployed into a valve seat of a previously implanted 45resent or docking station device (e.g., device 400). Cardiovascular implant device 600 can be delivered into the cardiovascular system via a catheter (i.e., transcatheter delivery) or can be surgically placed using transcatheter or surgical procedures known in the art. In some examples, device 600 can be delivered and/or implanted using the same catheter or surgical procedure that is used for a 45resent device (e.g., device 400). In other examples, device 600 can be delivered and/or implanted by a separate catheter or in a separate surgical procedure. Device 600 can be located in any vessel or chamber of heart H at a site in heart H where there is not naturally a valve (a "non-valve" site) or at a site where there is a natural valve (e.g., aortic valve AV, mitral valve MV, pulmonary valve PV, etc.). For example, FIG. 29 shows an example positioning of device 600 at aortic valve AV (a valve site). In contrast, FIG. 30 shows an example positioning of device 600 in inferior vena cava IVC (a non-valve site).

Frame 612 forms a main body of device 600. Frame 612 can be expandable. Frame 612 can have a wide variety of different shapes and sizes. As shown in FIG. 28, e.g., frame 612 is an annular or cylindrical mesh or lattice. Frame 612 has inner diameter 624 and outer diameter 626. Each of inner diameter 624 and outer diameter 626 can vary along a length of frame 612. Inner diameter 624 is a diameter of radially inner surface 625 of frame 612. Outer diameter 626 is a diameter of radially outer surface 627 of frame 612. Frame 612 can have any suitable length. For example, frame 612 may be approximately as long as valvular body 616. In other examples, frame 612 can be longer than valvular body 616. Frame 612 can press against or into tissue walls at the implant site or contour around anatomical structures of the cardiovascular system to set and maintain the position of device 600.

Frame 612 can be formed in a variety of ways, e.g., connecting individual wires together to form a mesh or lattice, braiding, cutting from a sheet and then rolling or otherwise forming into the shape of frame 612, molding, cutting from a cylindrical tube (e.g., cutting from a nitinol tube), other ways, or a combination of these. Frame 612 can be made from a highly flexible metal, metal alloy, or polymer. Examples of metals and metal alloys that can be used include, but are not limited to, nitinol and other shape-memory alloys, elgiloy, and stainless steel, but other metals and highly resilient or compliant non-metal materials can be used to make frame 612. All or a portion of frame 612 can be monolithically formed of any of these materials. These materials can allow frame 612 to be compressed to a small size, and then-when the compression force is released-frame 612 can self-expand back to its pre-compressed shape. Frame 612 can be expanded back to its pre-compressed shape due to the material properties of the material frame 112 is made out of and/or frame 612 can be expanded by inflation or expansion of a device positioned inside the frame. For example, frame 612 can be compressed such that frame 612 can fit into a delivery catheter. Frame 612 can also be made of other materials and can be expandable and collapsible in different ways, e.g., mechanically-expandable, balloon-expandable, self-expandable, or a combination of these.

Frame 612 extends between inflow end 618 and outflow end 620 of cardiovascular implant device 600. Inflow end 618 can be an end of device 600 that is relatively upstream of outflow end 620 with respect to a flow of blood along flow axis 630, as represented by arrow A in FIG. 28, when device 600 is implanted in a blood vessel or chamber of heart H. Accordingly, outflow end 620 is an end of device 600 that is relatively downstream of inflow end 618 with respect to a flow of blood along flow axis 630, as represented by arrow A in FIG. 28, when device 600 is implanted in a blood vessel or chamber of heart H. In the example shown in FIG. 29, outflow end 620 is positioned within aorta AT and inflow end 618 is positioned upstream at the site of aortic valve AV (facing left ventricle LV). In the example shown in FIG. 30, outflow end 620 is positioned near where inferior vena cava IVC opens into right atrium RA and inflow end 618 is positioned upstream within inferior vena cava IVC. Although inflow end 618 is defined as being relatively upstream of outflow end 620, it should be understood that other actual positions of inflow end 618 or outflow end 620 are possible depending on the location where device 600 is implanted.

Frame 612 is formed of a plurality of struts 622. Struts 622 make up the lattice or mesh of frame 612 and define openings (or cells) 628 therein. Struts 622 can be integrally formed. In some examples, all or a portion of struts 622 are monolithically formed from the same material. Openings 628 extend through frame 612 from inner surface 625 to outer surface 627. Each of openings 628 is bounded on one or more sides by ones of struts 622. Openings 628 can have any suitable shape or size, which can in turn be based on an overall shape or size of frame 612. In the example shown in FIG. 28, openings 628 are a combination of hexagonal and diamond shaped and arranged in circumferential rows around frame 612. In other examples, openings 628 can have any other regular or irregular polygonal or non-polygonal shape and pattern. In some examples, ones of openings 628 can have different shapes or sizes throughout frame 612. For example, FIG. 28 shows a first row of openings 628 (adjacent outflow end 620) with openings 628 that are hexagonal shaped and the remaining rows of openings 628 with openings 628 that are diamond shaped and smaller. In some examples, ones of openings 628 can be connected by gaps to adjacent ones of openings 628.

Central flow path 629 is an open channel through a central portion of annular frame 612. Central flow path 629 is defined by inner surface 625 of frame 612. Central flow path 629 extends from inflow end 618 to outflow end 620 such that device 600 is open at each end. Accordingly, blood flowing through and out of device 600 follows central flow path 629. More specifically, flow axis 630 is a longitudinal axis through device 600 along which blood flows as it passes or is directed through device 600 (e.g., in the direction indicated by arrow A in FIG. 28). In the example illustrated in FIG. 29 where device 600 is implanted at the site of aortic valve AV, device 600 can be closed (i.e., leaflets 631 of valvular body 616 can close) when heart H is in a diastolic phase. Blood flows from left atrium LA through mitral valve MV into left ventricle LV during the diastolic phase. During a systolic phase of heart H, device 600 can open. Blood flows from left ventricle LV through device 600 along flow axis 630 into aorta AT. In the example illustrated in FIG. 30 where device 600 is implanted in inferior vena cava IVC, device 600 can open when heart H is in a diastolic phase. Blood flows from inferior vena cava IVC and superior vena cava SVC into right atrium RA. The blood that flows from inferior vena cava IVC flows through device 600 along flow axis 630. During the diastolic phase, blood in right atrium RA flows through tricuspid valve TV and into right ventricle RV. During a systolic phase of heart H, device 600 can close. Blood is prevented from flowing (i.e., backflowing) from right atrium RA into inferior vena cava IVC by the closed device 600. A closed device 600 prevents any blood that regurgitates through the through tricuspid valve TV during the systolic phase from being forced into inferior vena cava IVC.

Cover 614 is a covering for one or more portions of frame 612. Cover 614 can be a fabric material, a polymer material, or other material. For example, cover 614 can be a material that promotes tissue ingrowth where device 600 contacts adjacent tissue walls of a vessel or chamber of heart H. Cover 614 can also form a seal to limit or prevent blood flow through portions of frame 612 that are covered by cover 614. Cover 614 can be attached to frame 612 by any suitable attachment means, such as by stitching, gluing, tying, etc. Cover 614 can be shaped and positioned in a variety of ways. In the example shown in FIG. 28, cover 614 is adjacent to inflow end 618. In some examples, cover 614 is near or adjacent an attachment region for valvular body 616. In other examples, cover 614 can be adjacent to outflow end 620 or at any location or locations between inflow end 618 and outflow end 620. In yet other examples, device 600 does not include cover 614.

Valvular body 616 is mounted within annular frame 612. More specifically, valvular body 616 is connected to inner surface 625 of frame 612. Valvular body 616 includes one or more leaflets 631. In the example shown in FIG. 28, there are three leaflets 631 (i.e., a tricuspid arrangement). In other examples, valvular body 616 can include more or fewer leaflets 631. The plurality of leaflets 631 are flexible and collapsible within frame 612 to regulate the flow of blood through device 600.

Once device 600 is implanted in cardiovascular system (e.g., in aorta AT at aortic valve AV as shown in FIG. 29 or in inferior vena cava IVC as shown in FIG. 30), circulating blood is delivered through device 600.

Cardiovascular implant device 600 can be manufactured at least in part using a PVD process as described above in reference to FIGS. 1-14 to improve the radiopacity of cardiovascular implant device 600. For example, cardiovascular implant device 600 can have a radiopaque coating applied to frame 612, similar to implantable medical device 60 as shown in and discussed in reference to FIGS. 5-7. Cardiovascular implant device 600 can also have a radiopaque layer and a super elastic layer applied to frame 612, similar to implantable medical device 90 as shown in and discussed in reference to FIGS. 8-10. Frame 612 of cardiovascular implant device 600 can also be manufactured out a ternary or quaternary metal alloy having radiopaque and super elastic properties, similar to implantable medical device 120 as shown in and discussed in reference to FIGS. 11-14. Alternatively, frame 612 of cardiovascular implant device 600 can be manufactured using a DFT wire having a radiopaque core and a super elastic sheath, similar to implantable medical device 260 shown in and discussed in reference to FIG. 15.

### CARDIOVASCULAR IMPLANT DEVICE 700 (not part of the claimed invention)

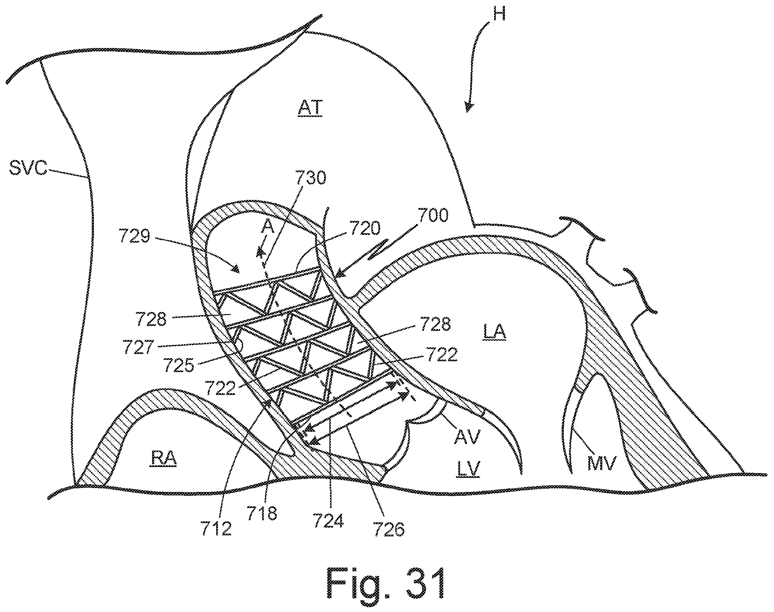

FIG. 31 is a sectional view of heart H illustrating an example positioning of cardiovascular implant device 700. As illustrated in FIG. 31, cardiovascular implant device 700 includes frame 712, inflow end 718, and outflow end 720. Frame 712 includes struts 722, inner diameter 724, inner surface 725, outer diameter 726, and outer surface 727, and defines openings 728, central flow path 729, and flow axis 730. FIG. 31 also shows device 700, heart H, right atrium RA, left atrium LA, left ventricle LV, superior vena cava SVC, mitral valve MV, aortic valve AV, and aorta AT.

Cardiovascular implant device 700 is an implantable device for use in a cardiovascular system. Cardiovascular implant device 700 is configured to be implanted in blood vessels or chambers of heart H. In the illustrated example, cardiovascular implant device 700 is a stent device. Cardiovascular implant device 700 can be delivered into the cardiovascular system via a catheter (i.e., transcatheter delivery) or can be surgically placed using transcatheter or surgical procedures known in the art. In some examples, device 700 can be delivered and/or implanted using the same catheter or surgical procedure that is used for an adjacent (or nearby) 49resent device (e.g., device 400) or a valve device (e.g., devices 500 and 600). In other examples, device 700 can be delivered and/or implanted by a separate catheter or in a separate surgical procedure. Device 700 can be located in any vessel or chamber of heart H. In some examples, device 700 is located at a site in heart H where there is not naturally a valve (a "non-valve" site). In other examples, device 700 is located near a site where there is a natural valve (e.g., near aortic valve AV, mitral valve MV, pulmonary valve PV, etc.). For example, FIG. 31 shows an example positioning of device 700 within aorta AT.

Frame 712 forms a main body of device 700. Frame 712 can be expandable. Frame 712 can have a wide variety of different shapes and sizes. As shown in FIG. 31, e.g., frame 712 is an annular or cylindrical mesh or lattice. Frame 712 has inner diameter 724 and outer diameter 726. Each of inner diameter 724 and outer diameter 726 can vary along a length of frame 712. Inner diameter 724 is a diameter of radially inner surface 725 of frame 712. Outer diameter 726 is a diameter of radially outer surface 727 of frame 712. Frame 712 can have any suitable length. Frame 712 can press against or into tissue walls at the implant site or contour (or extend) around anatomical structures of the cardiovascular system to set and maintain the position of device 700.

Frame 712 can be formed in a variety of ways, e.g., connecting individual wires together to form a mesh or lattice, braiding, cutting from a sheet and then rolling or otherwise forming into the shape of frame 712, molding, cutting from a cylindrical tube (e.g., cutting from a nitinol tube), other ways, or a combination of these. Frame 712 can be made from a highly flexible metal, metal alloy, or polymer. Examples of metals and metal alloys that can be used include, but are not limited to, nitinol and other shape-memory alloys, elgiloy, and stainless steel, but other metals and highly resilient or compliant non-metal materials can be used to make frame 712. All or a portion of frame 712 can be monolithically formed of any of these materials. These materials can allow frame 712 to be compressed to a small size, and then-when the compression force is released-frame 712 can self-expand back to its pre-compressed shape. Frame 712 can expand back to its pre-compressed shape due to the material properties of the material frame 712 is made out of and/or frame 712 can be expanded by inflation or expansion of a device positioned inside frame 712. For example, frame 712 can be compressed such that frame 712 can fit into a delivery catheter. Frame 712 can also be made of other materials and can be expandable and collapsible in different ways, e.g., mechanically-expandable, balloon-expandable, selfexpandable, or a combination of these.

Frame 712 extends between inflow end 718 and outflow end 720 of cardiovascular implant device 700. Inflow end 718 can be an end of device 700 that is relatively upstream of outflow end 720 with respect to a flow of blood along flow axis 730, as represented by arrow A in FIG. 31, when device 700 is implanted in a blood vessel or chamber of heart H. Accordingly, outflow end 720 is an end of device 700 that is relatively downstream of inflow end 718 with respect to a flow of blood along flow axis 730, as represented by arrow A in FIG. 31, when device 700 is implanted in a blood vessel or chamber of heart H. In the example shown in FIG. 31, outflow end 720 is positioned within aorta AT and inflow end 718 is positioned upstream nearer to the site of aortic valve AV. Although inflow end 718 is defined as being relatively upstream of outflow end 720, it should be understood that other actual positions of inflow end 718 or outflow end 720 are possible depending on the location where device 700 is implanted.

Frame 712 is formed of a plurality of struts 722. Struts 722 make up the lattice or mesh of frame 712 and define openings (or cells) 728 therein. Struts 722 can be integrally formed. In some examples, all or a portion of struts 722 are monolithically formed from the same material. Openings 728 extend through frame 712 from inner surface 725 to outer surface 727. Each of openings 728 is bounded on one or more sides by ones of struts 722. Openings 728 can have any suitable shape or size, which can in turn be based on an overall shape or size of frame 712. In the example shown in FIG. 31, openings 728 are triangular and arranged in circumferential rows around frame 712. In other examples, openings 728 can have any other regular or irregular polygonal or non-polygonal shape and pattern. In some examples, ones of openings 728 can have different shapes or sizes throughout frame 712. For example, FIG. 31 shows varying dimensions of openings 728 throughout frame 712. In some examples, ones of openings 728 can be connected by gaps to adjacent ones of openings 728.

Central flow path 729 is an open channel through a central portion of annular frame 712. Central flow path 729 is defined by inner surface 725 of frame 712. Central flow path 729 extends from inflow end 718 to outflow end 720 such that device 700 is open at each end. Accordingly, blood flowing through and out of device 700 follows central flow path 729. More specifically, flow axis 730 is a longitudinal axis through device 700 along which blood flows as it passes or is directed through device 700 (e.g., in the direction indicated by arrow A in FIG. 31). In the example illustrated in FIG. 31 where device 700 is implanted in aorta AT, aortic valve AV (or a prosthetic valve device, such as device 500) opens during a systolic phase of heart H. Blood flows from left ventricle LV through aortic valve AV into aorta AT. Within aorta AT, blood flows through device 700 along flow axis 730.

Although not shown in FIG. 31, device 700 can also include a cover, which can generally include the same structure and function as covers 414 and 514 described above.

Once device 700 is implanted in the cardiovascular system (e.g., in aorta AT as shown in FIG. 31), circulating blood passes through device 700.

Cardiovascular implant device 700 can be manufactured at least in part using a PVD process as described above in reference to FIGS. 1-14 to improve the radiopacity of cardiovascular implant device 700. For example, cardiovascular implant device 700 can have a radiopaque coating applied to frame 712, similar to implantable medical device 60 as shown in and discussed in reference to FIGS. 5-7. Cardiovascular implant device 700 can also have a radiopaque layer and a super elastic layer applied to frame 712, similar to implantable medical device 90 as shown in and discussed in reference to FIGS. 8-10. Frame 712 of cardiovascular implant device 700 can also be manufactured out a ternary or quaternary metal alloy having radiopaque and super elastic properties, similar to implantable medical device 120 as shown in and discussed in reference to FIGS. 11-14. Alternatively, frame 712 of cardiovascular implant device 700 can be manufactured using a DFT wire having a radiopaque core and a super elastic sheath, similar to implantable medical device 260 shown in and discussed in reference to FIG. 15.

### CARDIOVASCULAR IMPLANT DEVICE 800 (not part of the claimed invention)

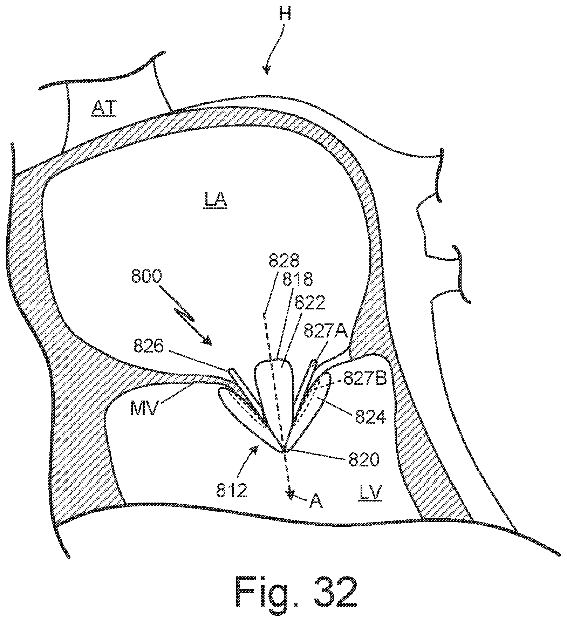

FIG. 32 is a sectional view of heart H illustrating an example positioning of cardiovascular implant device 800. As illustrated in FIG. 32, cardiovascular implant device 800 includes body 812, inflow end 818, and outflow end 820. Body 812 includes central spacer 822, paddles 824, clasps 826 (including first arm 827A and second arm 827B), and central longitudinal axis 828. FIG. 32 also shows device 800, heart H, left atrium LA, left ventricle LV, mitral valve MV, and aorta AT.

Cardiovascular implant device 800 is an implantable device for use in a cardiovascular system. Cardiovascular implant device 800 is configured to be implanted in blood vessels or chambers of heart H. In the illustrated example, cardiovascular implant device 800 is an edge-to-edge valve repair device. Cardiovascular implant device 800 can be delivered into the cardiovascular system via a catheter (i.e., transcatheter delivery) or can be surgically placed using transcatheter or surgical procedures known in the art. In some examples, device 800 can be delivered and/or implanted using the same catheter or surgical procedure that is used for an adjacent (or nearby) stent device (e.g., devices 700 and 800). In other examples, device 800 can be delivered and/or implanted by a separate catheter or in a separate surgical procedure. Device 800 can be located in any vessel or chamber of heart H. In particular, device 800 is located near a site where there is a natural valve (e.g., near mitral valve MV, a tricuspid valve, an aortic valve, a pulmonary valve, etc.). For example, FIG. 32 shows an example positioning of device 800 attached to mitral valve MV. Other examples can include device 800 attached to other natural valves, such as a tricuspid valve, an aortic valve, a pulmonary valve, etc.

Body 812 forms a main body of device 800. Body 812 can be formed in a variety of ways and can be made from a highly flexible metal, metal alloy, or polymer. Examples of metals and metal alloys that can be used include, but are not limited to, nitinol and other shape-memory alloys, elgiloy, and stainless steel, but other metals and highly resilient or compliant non-metal materials can be used to make body 812. All or a portion of body 812 can be monolithically formed of any of these materials. Body 812, including central spacer 822, paddles 824, and clasps 826, can have an expanded and a closed or collapsed configuration. For example, body 812 can be sized or collapsed to fit into a delivery catheter in a closed configuration. Body 812 can, in some examples, be expanded during an implantation procedure to attach device 800 to a natural valve of heart H.

Body 812 includes central spacer 822. Central spacer 822 forms a central portion of device 800. Central spacer 822 can be generally elongated, cylindrical, or tapered in shape. Central spacer 822 is configured to extend through an opening between leaflets of a natural valve of heart H and maintain a separation between sets of paddles 824 and clasps 826 that bridges the opening between the leaflets. Central longitudinal axis 828 extends longitudinally through central spacer 822.

Clasps 826 are elongated projections from body 812 that extend radially outward from central spacer 822 and central longitudinal axis 828. Clasps 826 include a respective first arm 827A and second arm 827B arranged in a U-shape or V-shape. First arms 827A of clasps 826 are configured to contact or press against a first side of the leaflets of the natural valve of heart H. In the example shown in FIG. 32, first arms 827A contact a side of the leaflets of mitral valve MV that faces left atrium LA. Second arms 827B (shown in dashed lines behind a portion of paddle 824 in FIG. 32) of clasps 826 are configured to contact or press against a second side of the leaflets of the natural valve of heart H. In the example shown in FIG. 32, second arms 827B contact a side of the leaflets of mitral valve MV that faces left ventricle LV. Pairs of first arms 827A and second arms 827B function together to grip the leaflet of leaflets of the natural valve of heart H.

Paddles 824 are paddle shaped or elongated and relatively widened and flattened projections from body 812 that extend radially outward from central spacer 822 and central longitudinal axis 828. Paddles 824 are configured to contact or press against second arms 827B of clasps 826. In the example shown in FIG. 32, paddles 824 are positioned on a side of mitral valve MV that faces left ventricle LV such that paddles 824 are within left ventricle LV. Each of paddles 824 has a 54resent54ndding one of clasps 826 for securing device 800 to the leaflets and holding the leaflets together around device 800. As such, pairs of paddles 824 and corresponding clasps 826 can have complimentary shapes and/or sizes so each pair fits together to grip the leaflet or leaflets. Paddles 824 and clasps 826 can be independently or cooperatively actuated to have different angles with respect to central longitudinal axis 828. An angle of paddles 824 with respect to central longitudinal axis 828 can be adjusted based on the desired amount of contact (i.e., pressure) between paddles 824 and clasps 826 at second arms 827B.

Body 812 extends between inflow end 818 and outflow end 820 of cardiovascular implant device 800. Inflow end 818 can be an end of device 800 that is relatively upstream of outflow end 820 with respect to a flow of blood parallel to central longitudinal axis 828, as represented by arrow A in FIG. 32, when device 800 is implanted in a blood vessel or chamber of heart H. Accordingly, outflow end 820 is an end of device 800 that is relatively downstream of inflow end 818 with respect to a flow of blood parallel to central longitudinal axis 828, as represented by arrow A in FIG. 32, when device 800 is implanted in a blood vessel or chamber of heart H. In the example shown in FIG. 32, outflow end 820 is positioned within left ventricle LV and inflow end 818 is positioned upstream within left atrium LA, such that left atrium LA pumps blood through mitral valve MV, around device 800, and into left ventricle LV. Although inflow end 818 is defined as being relatively upstream of outflow end 820, it should be understood that other actual positions of inflow end 818 or outflow end 820 are possible depending on the location where device 800 is implanted.

Although not shown in FIG. 32, device 800 can also include a cover, which can generally include the same structure and function as covers 314 and 614 described above.

Once device 800 is implanted in the cardiovascular system (e.g., attached to mitral valve MV as shown in FIG. 32), circulating blood passes around device 800.

Cardiovascular implant device 800 can be manufactured at least in part using a PVD process as described above in reference to FIGS. 1-14 to improve the radiopacity of cardiovascular implant device 800. For example, cardiovascular implant device 800 can have a radiopaque coating applied to body 812, similar to implantable medical device 60 as shown in and discussed in reference to FIGS. 5-7. Cardiovascular implant device 800 can also have a radiopaque layer and a super elastic layer applied to body 812, similar to implantable medical device 90 as shown in and discussed in reference to FIGS. 8-10. Body 812 of cardiovascular implant device 800 can also be manufactured out a ternary or quaternary metal alloy having radiopaque and super elastic properties, similar to implantable medical device 120 as shown in and discussed in reference to FIGS. 11-14. Alternatively, body 812 of cardiovascular implant device 800 can be manufactured using a DFT wire having a radiopaque core and a super elastic sheath, similar to implantable medical device 260 shown in and discussed in reference to FIG. 15.

Any of the various systems, devices, apparatuses, etc. in this disclosure can be sterilized (e.g., with heat, radiation, ethylene oxide, hydrogen peroxide, etc.) to ensure they are safe for use with patients, and the methods herein can comprise sterilization of the associated system, device, apparatus, etc. (e.g., with heat, radiation, ethylene oxide, hydrogen peroxide, etc.).

The treatment techniques, methods, steps, etc. described or suggested herein can be performed on a living animal or on a non-living simulation, such as on a cadaver, cadaver heart, anthropomorphic ghost, simulator (e.g., with the body parts, tissue, etc. being simulated), etc.

## Claims

1. A method of manufacturing an implantable medical device with radiopaque properties, the method comprising:
converting a first ingot of a radiopaque ternary alloy into a physical vapor deposition target, wherein the radiopaque ternary alloy is a nickeltitanium-platinum (NiTiPt) alloy;
depositing the radiopaque ternary alloy from the physical vapor deposition target onto a sacrificial substrate form using a physical vapor deposition process to create a radiopaque ternary alloy form;
removing the sacrificial substrate form from the radiopaque ternary alloy form; and
forming the implantable medical device from the radiopaque ternary alloy form,
wherein the implantable medical device is a shunt; and
wherein the shunt comprises a body comprising struts and openings between the struts, wherein the body comprises a central flow tube, a flow path and distal and proximal arms.

2. The method of claim 1, wherein the implantable medical device is between 50 micrometers and 250 micrometers thick.

3. The method of claim 1, wherein the implantable medical device is between 100 micrometers and 150 micrometers thick.

4. The method of any preceding claim, wherein the NiTiPt alloy has a stoichiometry of Ni_{42.7}Ti_{49.8}Pt₇.₅.

5. The method of any of claims 1 to 3, wherein the NiTiPt alloy has a stoichiometry of Ni_{40.2}Ti_{49.8}Pt₁₀.

6. The method of any of claims 1 to 3, wherein the NiTiPt alloy has a stoichiometry of Ni_{40.4}Ti_{49.6}Pt₁₀.

7. The method of any preceding claim, wherein forming the implantable medical device from the radiopaque ternary alloy form comprises: cutting the implantable medical device out of the radiopaque ternary alloy form.

8. The method of claim 7, wherein cutting the radiopaque ternary alloy form comprises: laser cutting the implantable medical device out of the radiopaque ternary alloy form.

9. The method of claim 7, and further comprising: shape setting and/or heat treating the implantable medical device.

10. The method of claim 7, and further comprising:
surface finishing the implantable medical device using a surface finishing process; and/or
sterilizing the implantable medical device.

11. The method of claim 10, wherein the surface finishing process is chosen from the group consisting of mechanical polishing, chemical etching, chemical polishing, electropolishing, and combinations thereof.

12. The method of any preceding claim, wherein converting the first ingot of the radiopaque ternary alloy into a physical vapor deposition target comprises:
melting the first ingot into a sheet of radiopaque ternary alloy; and
rolling the sheet into a cylindrical physical vapor deposition target.

13. An implantable medical device with radiopaque properties, wherein the implantable medical device is a shunt, and the shunt comprises:
a body comprising struts and openings between the struts;
wherein the body comprises a central flow tube, a flow path and distal and proximal arms;
wherein the implantable medical device is manufactured using a physical vapor deposition process; and
wherein the implantable medical device is made from a nickel-titanium-platinum (NiTiPt) alloy.

14. The implantable medical device of claim 14, wherein the NiTiPt alloy has a stoichiometry of:
a) Ni_{42.7}Ti_{49.8}Pt_{7.5};
b) Ni_{40.2}Ti_{49.8}Pt₁₀; or
c) Ni_{40.4}Ti_{49.6}Pt₁₀.

## Patentansprüche

1. Verfahren zum Herstellen einer implantierbaren medizinischen Vorrichtung mit strahlungsundurchlässigen Eigenschaften, wobei das Verfahren Folgendes umfasst:
Umwandeln eines ersten Barrens einer strahlungsundurchlässigen ternären Legierung in ein physikalisches Gasphasenabscheidungsziel, wobei die strahlungsundurchlässige ternäre Legierung eine Nickel-Titan-Platin(NiTiPt)-Legierung ist;
Abscheiden der strahlungsundurchlässigen ternären Legierung aus dem physikalischen Gasphasenabscheidungsziel auf eine Opfersubstratform unter Verwendung eines physikalischen Gasphasenabscheidungsprozesses, um eine strahlungsundurchlässige ternäre Legierungsform zu erzeugen;
Entfernen der Opfersubstratform von der strahlungsundurchlässigen ternären Legierungsform und
Bilden der implantierbaren medizinischen Vorrichtung aus der strahlungsundurchlässigen ternären Legierungsform,
wobei die implantierbare medizinische Vorrichtung ein Shunt ist und
wobei der Shunt einen Körper umfasst, der Streben und Öffnungen zwischen den Streben umfasst, wobei der Körper ein zentrales Durchflussrohr, einen Durchflussweg und distale und proximale Arme umfasst.

2. Verfahren nach Anspruch 1, wobei die implantierbare medizinische Vorrichtung zwischen 50 Mikrometer und 250 Mikrometer dick ist.

3. Verfahren nach Anspruch 1, wobei die implantierbare medizinische Vorrichtung zwischen 100 Mikrometer und 150 Mikrometer dick ist.

4. Verfahren nach einem der vorhergehenden Ansprüche, wobei die NiTiPt-Legierung eine Stöchiometrie von Ni_{42,7}Ti_{49,8}Pt_{7,5} aufweist.

5. Verfahren nach einem der Ansprüche 1 bis 3, wobei die NiTiPt-Legierung eine Stöchiometrie von Ni_{40,2}Ti_{49,8}Pt₁₀ aufweist.

6. Verfahren nach einem der Ansprüche 1 bis 3, wobei die NiTiPt-Legierung eine Stöchiometrie von Ni_{40,4}Ti_{49,6}Pt₁₀ aufweist.

7. Verfahren nach einem der vorhergehenden Ansprüche, wobei das Bilden der implantierbaren medizinischen Vorrichtung aus der strahlungsundurchlässigen ternären Legierungsform Folgendes umfasst: Schneiden der implantierbaren medizinischen Vorrichtung aus der strahlungsundurchlässigen ternären Legierungsform.

8. Verfahren nach Anspruch 7, wobei das Schneiden der strahlungsundurchlässigen ternären Legierungsform Folgendes umfasst: Laserschneiden der implantierbaren medizinischen Vorrichtung aus der strahlungsundurchlässigen ternären Legierungsform.

9. Verfahren nach Anspruch 7, das ferner Folgendes umfasst: Formfestigen und/oder Wärmebehandeln der implantierbaren medizinischen Vorrichtung.

10. Verfahren nach Anspruch 7, das ferner Folgendes umfasst:
Oberflächenveredeln der implantierbaren medizinischen Vorrichtung unter Verwendung eines Oberflächenveredelungsprozesses und/oder
Sterilisieren der implantierbaren medizinischen Vorrichtung.

11. Verfahren nach Anspruch 10, wobei der Oberflächenveredelungsprozess ausgewählt ist aus der Gruppe bestehend aus mechanischem Polieren, chemischem Ätzen, chemischem Polieren, Elektropolieren und Kombinationen davon.

12. Verfahren nach einem der vorhergehenden Ansprüche, wobei das Umwandeln des ersten Barrens der strahlungsundurchlässigen ternären Legierung in ein physikalisches Gasphasenabscheidungsziel Folgendes umfasst:
Schmelzen des ersten Barrens in ein Blech aus strahlungsundurchlässiger ternärer Legierung und
Rollen des Blechs zu einem zylindrischen physikalischen Gasphasenabscheidungsziel.

13. Implantierbare medizinische Vorrichtung mit strahlungsundurchlässigen Eigenschaften, wobei die implantierbare medizinische Vorrichtung ein Shunt ist und der Shunt Folgendes umfasst:
einen Körper, der Streben und Öffnungen zwischen den Streben umfasst;
wobei der Körper ein zentrales Strömungsrohr, einen Strömungsweg und distale und proximale Arme umfasst;
wobei die implantierbare medizinische Vorrichtung unter Verwendung eines physikalischen Gasphasenabscheidungsprozesses hergestellt wird und
wobei die implantierbare medizinische Vorrichtung aus einer Nickel-Titan-Platin(NiTiPt)-Legierung hergestellt ist.

14. Implantierbare medizinische Vorrichtung nach Anspruch 14, wobei die NiTiPt-Legierung eine der folgenden Stöchiometrien aufweist:
a) Ni_{42,7}Ti_{49,8}Pt_{7,5};
b) Ni_{40,2}Ti_{49,8}Pt₁₀ oder
c) Ni_{40,4}Ti_{49,6}Pt₁₀.

## Revendications

1. Procédé de fabrication d'un dispositif médical implantable ayant des propriétés radio-opaques, le procédé comprenant :
la conversion d'un premier lingot d'un alliage ternaire radio-opaque en une cible de dépôt physique en phase vapeur, l'alliage ternaire radio-opaque étant un alliage nickel-titane-platine (NiTiPt) ;
le dépôt de l'alliage ternaire radio-opaque de la cible de dépôt physique en phase vapeur sur une forme de substrat sacrificiel à l'aide d'un procédé de dépôt physique en phase vapeur pour créer une forme d'alliage ternaire radio-opaque ;
le retrait de la forme de substrat sacrificiel à partir de la forme d'alliage ternaire radio-opaque ; et
la formation du dispositif médical implantable à partir de la forme d'alliage ternaire radio-opaque,
le dispositif médical implantable étant une dérivation ; et
la dérivation comprenant un corps comprenant des entretoises et des ouvertures entre les entretoises, le corps comprenant un tube d'écoulement central, une voie d'écoulement et des bras distal et proximal.

2. Procédé selon la revendication 1, le dispositif médical implantable ayant une épaisseur comprise entre 50 micromètres et 250 micromètres.

3. Procédé selon la revendication 1, le dispositif médical implantable ayant une épaisseur comprise entre 100 micromètres et 150 micromètres.

4. Procédé selon l'une quelconque des revendications précédentes, l'alliage NiTiPt ayant une stœchiométrie de Ni_{42,7}Ti_{49,8}Pt_{7,5}.

5. Procédé selon l'une quelconque des revendications 1 à 3, l'alliage NiTiPt ayant une stœchiométrie de Ni_{40,2}Ti_{49,8}Pt₁₀.

6. Procédé selon l'une quelconque des revendications 1 à 3, l'alliage NiTiPt ayant une stœchiométrie de Ni_{40,4}Ti_{49,6}Pt₁₀.

7. Procédé selon l'une quelconque des revendications précédentes, la formation du dispositif médical implantable à partir de la forme d'alliage ternaire radio-opaque comprenant : la découpe du dispositif médical implantable hors de la forme d'alliage ternaire radio-opaque.

8. Procédé selon la revendication 7, la découpe de la forme d'alliage ternaire radio-opaque comprenant : la découpe au laser du dispositif médical implantable hors de la forme d'alliage ternaire radio-opaque.

9. Procédé selon la revendication 7, et comprenant en outre : le réglage de forme et/ou le traitement thermique du dispositif médical implantable.

10. Procédé selon la revendication 7, et comprenant en outre :
la finition de surface du dispositif médical implantable à l'aide d'un processus de finition de surface ; et/ou
la stérilisation du dispositif médical implantable.

11. Procédé selon la revendication 10, le processus de finition de surface étant choisi dans le groupe constitué par le polissage mécanique, la gravure chimique, le polissage chimique, l'électropolissage et des combinaisons de ceux-ci.

12. Procédé selon l'une quelconque des revendications précédentes, la conversion du premier lingot de l'alliage ternaire radio-opaque en une cible de dépôt physique en phase vapeur comprenant :
la fusion du premier lingot en une feuille d'alliage ternaire radio-opaque ; et
le laminage de la feuille en une cible de dépôt physique en phase vapeur cylindrique.

13. Dispositif médical implantable ayant des propriétés radio-opaques, le dispositif médical implantable étant une dérivation, et la dérivation comprenant :
un corps comprenant des entretoises et des ouvertures entre les entretoises ;
le corps comprenant un tube d'écoulement central, une voie d'écoulement et des bras distal et proximal ;
le dispositif médical implantable étant fabriqué à l'aide d'un processus de dépôt physique en phase vapeur ; et
le dispositif médical implantable étant constitué d'un alliage nickel-titane-platine (NiTiPt).

14. Dispositif médical implantable selon la revendication 14, l'alliage NiTiPt ayant une stœchiométrie de :
a) Ni_{42,7}Ti_{49,8}Pt_{7,5};
b) Ni_{40,2}Ti_{49,8}Pt₁₀; or
c) Ni_{40,4}Ti_{49,6}Pt₁₀.
